# EUROPEAN PATENT SPECIFICATION

(11) **EP 0 521 099 B1**
(45) Date of publication and mention of the grant of the patent: **29.12.1997**
(21) Application number: 91907292.6
(22) Date of filing: 21.03.1991
(51) Int. Cl.: C07F 13/00, B01J 31/00, B01J 31/18

(54) **CHIRAL CATALYSTS AND EPOXIDATION REACTIONS CATALYZED THEREBY**
CHIRALE KATALYSATOREN UND DADURCH KATALYSIERTE EPOXYDIERUNGSREAKTIONEN
CATALYSEURS CHIRAUX ET REACTIONS D'EPOXYDATION CATALYSEES PAR CE MOYEN

(30) Priority: 21.03.1990 US 496992
(43) Date of publication of application: 07.01.1993
(73) Proprietor: RESEARCH CORPORATION TECHNOLOGIES, INC., Tucson Arizona 85710-2815 (US)
(72) Inventor: JACOBSEN, Eric, N., Mahomet, IL 61853 (US); ZHANG, Wei, Urbana, IL 61801 (US)
(74) Representative: Vossius, Volker, Dr.
(86) International application number: US9101897
(87) International publication number: WO9114694

(56) References cited:
- EP-A- 0 222 167
- FR-A- 2 588 265
- Journal of the American Chemical Society, Volume 108, No. 9, issued 30 April 1986, SRINIVASAN et al. "Epoxidation of Olefins with Cationic (Salen)MnIII Complexes. The Modulation of Catalytic Activity by Substituents", see entire document.
- CHEMICAL ABSTRACTS, Volume 104, No. 5, issued 03 February 1986 (Columbus, Ohio, US), MASAFUMI et al. "Analysis of the induction period and proposal of mechanism for the catalysis of 3-methylindole oxygenation by two isomers of N,N'-(1,2-cyclohexylene)bis(3-tert-butylsalicyl=ideneaminato)cobalt(II)", see page 513, column 1, Abstract No. 33700x, Chem. Pharm. Bull. 1985, 33(6), 2195-203 (Eng.).
- Journal of the American Chemical Society, Volume 112, No. 7, issued 28 March 1990, WEI ZHANG et al. "Enantioselective Epoxidation of Unfunctionalized Olefins Catalyzed by (Salen) manganese Complexes", see entire document.
- Chemical Society of Japan, Bulletin, Volume 56, issued January 1983, HAJIME KANATOMI "The Dehydrogenation of Nickel(II) Chelates of rac- and meso-2,2'-(1.2,-Diphenylethylene) bis(iminomethylene)=diphenol and related compounds", see pages 99, 103 and 104.
- Inorganic Chemistry, Volume 23, No. 16, issued 01 August 1984, MINELLI et al. "95 MoNMR Measurements of Dioxomolybdenum(VI) Complexes. 3. Inverse Halogen Dependence of the Molybdenum Chemical Shifts of (MoO2)2+ Complexes", see entire document.
- Chemical Letters, No. 9, issued September 1986, NAKAJIMA et al. "Asymmetric Oxidation of Sulfides to Sulfoxides by Organic Hydroperoxides with Optically Active Schiff Base-Oxovanadium(IV) Catalysts", see pages 1483 and 1484. Also see figure 1.
- Chemical Society of Japan, Bulletin, Volume 60, issued December 1987, OKAMOTO et al. "Effect of Catalyst on the Oxygenation of Styrene with BH4 and Molecular Oxygen", see entire document.

## Description

The present invention relates to the field of asymmetric catalysis. More particularly, the invention relates to the field of organometallic catalysts useful for enantioselectively epoxidizing prochiral olefins.

Several advances in catalysis of asymmetric group transfer have occurred in recent years. One such advance has been the discovery by K.B. Sharpless et al. of the epoxidation of allylic alcohols which provides access to enantiomerically pure synthetic building blocks. Unfortunately, Sharpless catalysis requires the presence of a specific functional group, namely an allylic alcohol, on the olefin to be epoxidized. Naturally, this requirement severely limits the variety of olefins which can be so epoxidized.

Some success has been achieved in asymmetric catalysis of unfunctionalized olefins. For example, K.B. Sharpless reported in 1988 that certain cinchona alkaloid derivatives were effective ligands in the osmium-catalyzed asymmetric dihydroxylation of transstilbene and various other olefins. This method provides a practical route to certain chiral diols, although cis olefins afford poor results.

There currently exists no practical catalytic method for the asymmetric epoxidation of unfunctionalized olefins. Some progress has been made in this area through the use of chiral porphyrin complexes. In particular, J.T. Groves et al. reported in 1983 the asymmetric epoxidation of styrene by a chiral iron porphyrin catalyst. Unfortunately, the Groves system suffers several disadvantages, namely, the porphyrin catalyst is relatively difficult to prepare, the system employs an impractical oxidant (iodosylmesitylene), proceeds to low substrate conversion, is limited to styrene derivatives, and achieves enantomeric excess (ee) values of less than about 50 percent. Srinivasan et al., Journal of the American Chemical Society, Vol. 108, No. 9, issued 30 April 1986, "Epoxidation of Olefins with Cationic (Salen)Mn^{III} Complexes. The Modulation of Catalytic Activity by Substituents" describe that Cationic manganese(III) complexes of the salen ligand are effective catalysts for the epoxidation of various olefins. Stereospecific epoxidation is achieved with *trans*-olefins such as (E)-2-hexene and (E)-β-methylstyrene, *cis*-Olefins produce high yields of *cis*-epoxides which contain minor amounts of the corresponding trans isomer.

Given the broad synthetic utility of epoxides, a simple, reliable, and practical procedure for asymmetric epoxidation of simple olefins is clearly desirable.

### SUMMARY OF THE INVENTION

Briefly stated, the present invention relates to chiral catalysts as well as a method of using said catalysts for enantioselectively epoxidizing a prochiral olefin.

In accordance with a first aspect of the invention, the chiral catalyst has the general formula (I): 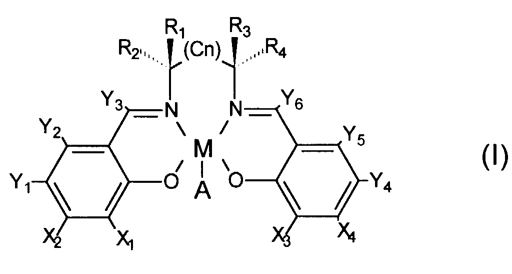 wherein M is a transition metal ion, A is an anion, and n is either 0, 1, or 2. At least one of X1 or X2 is selected from the group consisting of aryls, primary alkyls, secondary alkyls and tertiary alkyls, hetero atoms, and alkyl groups bearing hetero atoms; where at least one of X3 or X4 is selected from the same group. Y1, Y2, Y4 and Y5 are independently selected from the group consisting of hydrogen, halides, alkyls, aryls, alkyl groups bearing hetero atoms, alkoxy and nitro and Y3 and Y6 are independently selected from the group consisting of hydrogen, halides, alkyls, aryls and alkyl groups bearing hetero atoms. Also, at least one of R1, R2, R3 and R4 is selected from a first group consisting of H, CH₃, C₂H₅, and primary alkyls. Furthermore, if R1 selected from said first group, then R2 and R3 are selected from a second group consisting of aryl groups, secondary alkyls and tertiary alkyls and alkyl groups bearing hetero atoms. If R2 is selected from said first group, then R1 and R4 are selected from said second group. If R3 is selected from said first group, then R1 and R4 are selected from said second group. If R4 is selected from said first group, then R2 and R3 are selected from said second group. The carbon atom(s) in the (Cₙ) portion have substituent selected from the group consisting of hydrogen, alkyl, aryl and hetero atoms.

In accordance with a second aspect of the invention, the chiral catalyst has the general formula (II) : 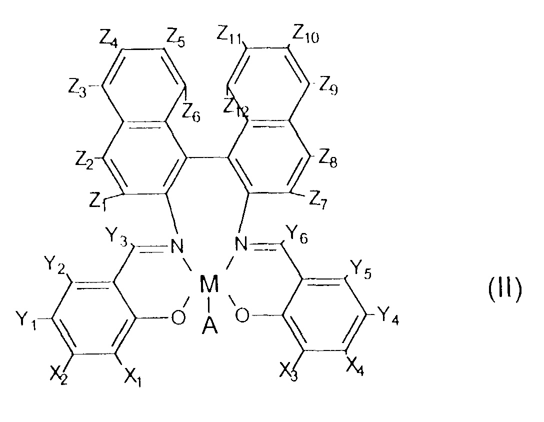 wherein M is a transition metal ion and A is an anion; where at least one of X1 or X2 is selected from the group consisting of aryls, primary alkyls, secondary alkyls, tertiary alkyls, hetero atoms and alkyl groups bearing hetero atoms; where at least one of X3 or X4 is selected from the group consisting of aryls, primary alkyls, secondary alkyls, tertiary alkyls, hetero atoms and alkyl groups bearing hetero atoms; and where Y1, Y2, Y3, Y4, Y5, Y6, Z1, Z2, Z3, Z4, Z5, Z6, Z7, Z8, Z9, Z10, Z11, and Z12 are independently selected from the group consisting of hydrogen, halides, alkyls, aryls, alkyl groups bearing hetero atoms, and hetero atoms.

In accordance with a third aspect of the invention, the chiral catalyst has the general formula (III) : 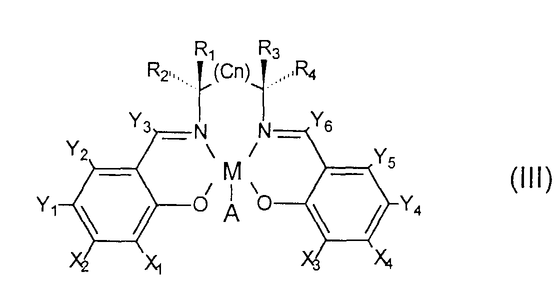 where M is a transition metal ion and A is an anion; where n is either 0, 1, or 2; where at least one of X1 or X2 is selected from the group consisting of aryls, primary alkyls, secondary alkyls, tertiary alkyls, hetero atoms and alkyl groups bearing hetero atoms; where at least one of X3 or X4 is selected from the group consisting of aryls, primary alkyls, secondary alkyls, tertiary alkyls, hetero atoms and alkyl groups bearing hetero atoms; where at least one of Y1 or Y2 is selected from the group consisting of aryls, primary alkyls, secondary alkyls, tertiary alkyls, hetero atoms and alkyl groups bearing hetero atoms; where at least one of Y4 or Y5 is selected from the group consisting of aryls, primary alkyls, secondary alkyls, tertiary alkyls, hetero atoms and alkyl groups bearing hetero atoms; where Y3 and Y6 are independently selected from the group consisting of hydrogen and primary alkyl groups; where either one or two of R1, R2, R3 and R4 is hydrogen; where, if R1 is hydrogen, then R3 is a primary alkyl; where, if R2 is hydrogen, then R4 is a primary alkyl; where, if R3 is hydrogen, then R1 is a primary alkyl; and where, if R4 is hydrogen, then R2 is a primary alkyl; and where the carbon(s) in the (Cₙ) portion have substituents selected from the group consisting of hydrogen, alkyl, aryl and hetero atoms.

In accordance with the fourth aspect of the present invention, the chiral catalyst has the general formula (IV) : 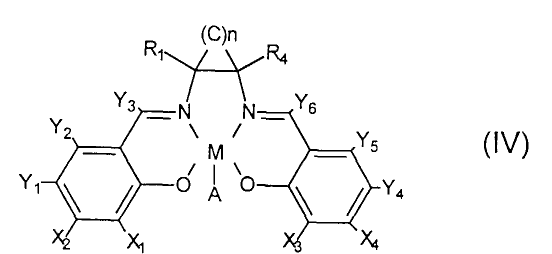 where M is a transition metal ion and A is an anion; where n is either 3, 4, 5 or 6; where at least one of X1 or X2 is selected from the group consisting of aryls, primary alkyls, secondary alkyls, tertiary alkyls, hetero atoms and alkyl groups bearing hetero atoms; where at least one of X3 or X4 is selected from the group consisting of aryls, primary alkyls, secondary alkyls, tertiary alkyls, hetero atoms and alkyl groups bearing hetero atoms; where at least one of Y1 or Y2 is selected from the group consisting of aryls, primary alkyls, secondary alkyls, tertiary alkyls, hetero atoms and alkyl groups bearinq hetero atoms; where at least one of Y4 or Y5 is selected from the group consisting of aryls, primary alkyls, secondary alkyls, tertiary alkyls, hetero atoms and alkyl groups bearing hetero atoms; where Y3, and Y6 are independently selected from the group consisting of hydrogen and primary alkyl groups; where R1 and R4 are trans to each other and are selected from the group consisting of primary alkyls and hydrogen; and where the carbons in the (C)ₙ portion have substituents selected from the group consisting of hydrogen, alkyl, aryl, and heteroatoms.

In accordance with the method aspect of the invention, the prochiral olefin, an oxygen atom source, and the chiral catalyst of any one of the four aspects of the invention are reacted under such conditions and for such time as is needed to epoxidize said olefin.

The present invention has provided certain advantages. First, the catalysts of the present invention provide a means for catalyzing the enantioselective epoxidation of mono, di, and tri-substituted olefins without the need for a specialized functional group on the olefin to interact with the catalyst. In other words, the catalysts of the present invention are particularly suited for catalyzing the asymmetric epoxidation of unfunctionalized olefins. This is in contrast to the prior art catalysts, such as the Sharpless catalyst, referred to above.

Second, the preferred catalysts of the invention show remarkable enantioselectivity in catalyzing the epoxidation of cis, disubstituted olefins. See Example 5 below, where an ee value of 85% was obtained with cis-β-methylstyrene when catalyzed with the most preferred embodiment of the first aspect. See also, the ee value for Example 12 which uses the most preferred catalyst of the fourth aspect of the present invention. As noted above, prior art catalysts have not provided ee values over 40% for cis, disubstituted olefins.

Third, the catalysts of the present invention are relatively easy to synthesize, particularly as compared to the porphyrin systems disclosed in the prior art.

The present invention, together with attendant objects and advantages, will be best understood with reference to the detailed description below read in conjunction with the accompanying figures.

### BRIEF DESCRIPTION OF THE DRAWINGS

FIGURE 1 shows the generalized 2-dimensional structure of a catalyst of the first aspect of the present invention.

FIGURE 2 shows the 2-dimensional structure of the most preferred catalyst of the first aspect of the present invention.

FIGURE 3 shows the generalized 2-dimensional structure of the catalyst of the second aspect of the present invention.

FIGURE 4 is a computer generated 3-dimensional view of the most preferred catalyst of the present invention in its proposed oxo-intermediate state.

FIGURES 5A-5C are views similar to FIGURE 4 illustrating the steric hindrance believed to be responsible for the high enantioselectivity observed in the epoxidation of one of the preferred substrates by the preferred catalysts of the present invention.

FIGURE 6 shows the generalized 2-dimensional structure of the catalyst of the third aspect of the present invention.

FIGURE 7 shows the generalized 2-dimensional structure of the catalyst of the fourth aspect of the present invention.

FIGURE 8 shows the 2-dimensional structure of the preferred catalyst of the fourth aspect of the present invention.

FIGURE 9 is a 2-dimensional representation of the theorized favored approach of a prochiral olefin to a preferred catalyst of the first aspect of the invention.

FIGURE 10 is a 2-dimensional representation of the theorized favored approach of a prochiral olefin to a preferred catalyst of the second aspect of the invention.

FIGURE 11 shows 2-dimensional structures for various embodiments of the present invention with the numbering system used in Examples 8-16.

### DETAILED DESCRIPTION OF THE PREFERRED EMBODIMENTS

As noted above, the present invention is a chiral catalyst as well as a method of using said catalyst for enantioselectively epoxidizing prochiral olefins.

### The First Aspect of the Invention

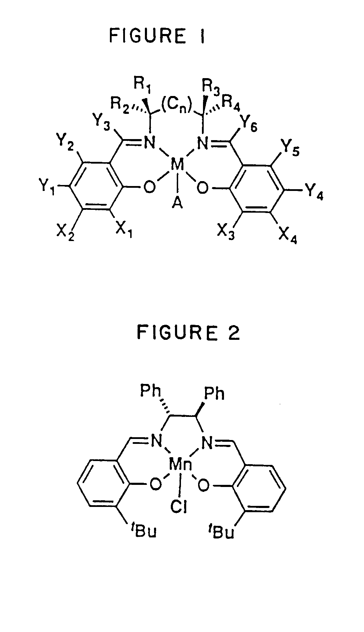

FIGURE 1 shows the structure of the preferred chiral catalyst of the first aspect of the present invention.

The preferred catalysts of the present invention are salen derivative-based complexes of a metal ion. The term "salen" is used herein to refer to those ligands typically formed through a condensation reaction of two molecules of a salicylaldehyde derivative with one molecule of a diamine derivative. While salen ligands are formed from ethylenediamine derivatives, propylene and butylene diamines may also be used to give analogous salpn and salbn derivatives. Salen derivatives are preferred and their general structure is shown in FIGURE 1 where n is 0 and is shown in FIGURE 2.

As seen in FIGURE 1, the two nitrogens and the two oxygens are oriented toward the center of the salen ligand and thus provide a complexing site for the transition metal ion M. Preferably, this metal ion is selected from the group consisting of Mn, Cr, Fe, Ni, Co, Ti, V, Ru, and Os. More preferably, the transition metal ion is selected from the group consisting of Mn, Cr, Fe, Ni, and Co. Most preferably, the metal ion is Mn.

The selection of the anion, A, is not seen to be critical to the performance of the catalyst. Preferably, the anion is selected from the group consisting of PF₆, (aryl)₄, BF₄,B(aryl)₄, halide, acetate, triflate, tosylate, with halide or PF₆ being more preferred, and chloride being most preferred.

FIGURE 1 also shows the many sites available for substitution on the salen ligand. Of these sites, it is believed that R1, R2, R3, R4, and X1, X2, X3, X4, Y3 and Y6 are the most important in this first aspect of the invention.

According to the first aspect of the invention, at least one of the X1 and X2 sites, and at least one of the X3 and X4 sites include a substituent selected from the group consisting of primary, secondary or tertiary alkyl groups, aryl groups, hetero atoms and alkyl groups bearing heteroatom substituents such as alkoxy or halide. For reasons to be discussed below, these will be referred to as "blocking" substituents. Preferably, it is the X1 and X3 sites which bear one of these blocking substituents. More preferably, X1 and X3 bear the same substituent, which substituent is most preferably a tertiary alkyl group, such as tertiary butyl. Preferably, When X1 and X3 bear the blocking substituent, then X2 and X4 can be selected from a group of non-blocking substituents such as H, CH₃, C₂H₅, and primary alkyls, most preferably, H. Alternatively, either three or four of X1, X2, X3, and X4 can be selected from the group of blocking substituents.

According to this first aspect of the invention, at least one and no more than two of R1, R2, R3 and R4 are selected from a first group consisting of H, CH₃, C₂H₅, and primary alkyls. For convenience, and consistent with the present theory to be discussed below, this first group will be referred to as the non-blocking group. If R1 is selected from the non-blocking group, then R2 and R3 are selected from a second group consisting of aryls, secondary alkyls, and tertiary alkyls and alkyl groups bearing hetero atoms. This second group will be referred to as the blocking group. If R2 is selected from the non-blocking group, then R1 and R4 are selected from the blocking group. Likewise, if R3 is selected from the non-blocking group, then R1 and R4 are selected from the blocking group. Finally, if R4 is selected from the non-blocking group, then R2 and R3 are selected from the blocking group.

Stated in other terms, this first aspect of the invention requires that, of the four sites available for substitution on the two carbon atoms adjacent to nitrogen, either one or two of these will include a substituent from the non-blocking group. The invention also requires that the remaining sites include a substituent from the blocking group. In addition, it is a requirement that there not be two non-blocking substituents on the same carbon, and that there not be two non-blocking substituents on the same side on the two different carbons, i.e. not cis across the nitrogen.

Stated in yet another way, if there is only one non-blocking substituent, that non-blocking substituent can be on any one of the four substitution sites, R1, R2, R3, and R4, and the other three sites must include a blocking substituent. If, on the other hand, there are two non-blocking substituents, then they must be on different carbon atoms, and they must be trans to each other.

Preferably, the non-blocking substituent is either hydrogen or methyl, most preferably, hydrogen. Preferably, the blocking substituent is either a phenyl group or a tertiary butyl group, most preferably a phenyl group.

The substituents on the Y3 and Y6 sites affect the conformation of the ligand and thus have an influence on enantioselectivity in the epoxidation. Preferably, Y3 and Y6 are hydrogen, methyl, alkyl, or aryl. More preferably, they are hydrogen or methyl. Most preferably, they are hydrogen.

The Y1, Y2, Y4, and Y5 sites are seen to be less critical. Preferably, these sites are occupied by hydrogen, although these sites may also be occupied by substituents independently selected from the group consisting of hydrogen, halides, alkyls, aryls, alkoxy groups, nitro groups and alkyl groups bearing hetero atoms.

FIGURE 2 shows the structure of the most preferred catalyst of this first aspect of the present invention. As can be seen, the most preferred substituent at X1 and X3 is a t-butyl group. Also, it is most preferred for the R1 and R4 sites to have the same blocking group, namely a phenyl group. In addition, it is most preferred to have the R2 and R3 sites occupied by a hydrogen. Finally, it is most preferred that the X2, X4, Y1, Y2, Y3, Y4, Y5, and Y6 sites are also all occupied by a hydrogen.

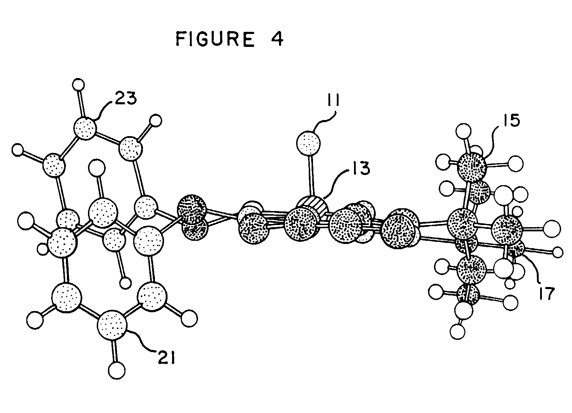

While not wishing to be bound by any particular theory, the following mechanism has been proposed to explain the remarkable enantioselectivity of the catalysts of the first aspect of the present invention. Referring to FIGURE 4, which is a 3-dimensional view of the R,R enantiomer of the most preferred catalyst in its proposed oxo-intermediate state, it is seen that, with important exceptions, the salen ligand assumes a generally planar conformation with the oxygen atom 11 being complexed with the Manganese ion 13 and aligned on an axis generally perpendicular to this plane. The exceptions are the tert-butyl blocking groups attached at the X1 and X3 sites 15 and 17 respectively, and the phenyl blocking groups attached at the R1 and R3 sites 21 and 23 respectively. Although hard to depict in two dimensions, the phenyl blocking group 23 at R4 is behind the phenyl blocking group 21 at R1, while the R4 phenyl blocking group 23 is substantially above the plane of the catalyst and the R1 phenyl blocking group 21 is substantially below the plane of the catalyst.

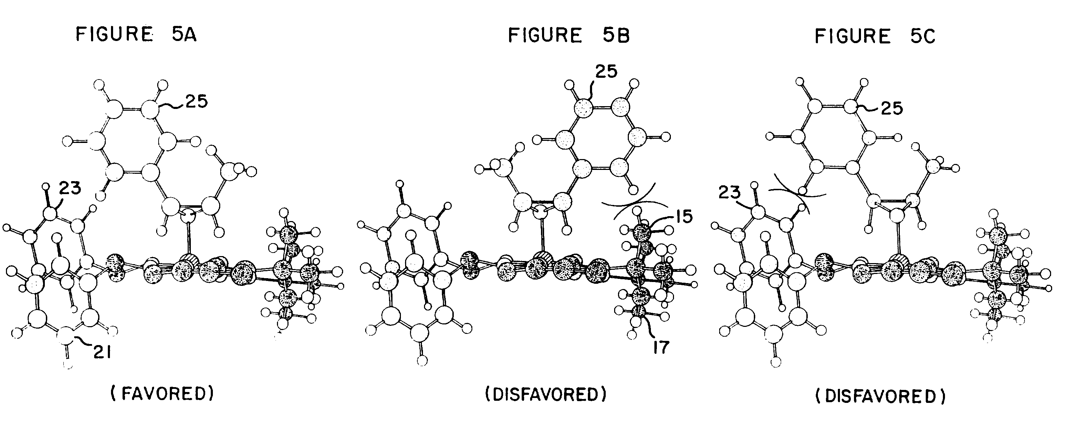

FIGURES 5A-5C show the different transition orientations possible for a cis-disubstituted olefin, namely cis-β-methyl styrene, which are possible during epoxidation of the double bond.

FIGURE 5A shows the favored orientation, i.e. the orientation with the least steric hindrance between the olefin and the blocking groups of the catalyst. This orientation results when the double bond approaches the oxygen atom from the front (as shown). This orientation results in the formation of the 1R,2S enantiomer of the cis-β-methyl styreneoxide.

FIGURE 5B shows an orientation wherein methyl styrene has been rotated 180 degrees thus bringing the phenyl group of the styrene closer to the t-butyl groups 15 and 17 at the X1 and X3 positions. It is expected that steric hindrance between the phenyl group of the styrene 25 and the t-butyl groups 15 and 17 would disfavor this orientation.

FIGURE 5C shows an orientation resulting from the double bond approaching the oxygen atom from behind (as shown). This orientation results in the formation of the 1S,2R enantiomer of the cis-β-methyl styreneoxide. In this orientation the phenyl group of the styrene 25 is closer to the phenyl group 23 on the R4 site. Steric hindrance between these two phenyl groups would thus disfavor this approach from behind the oxygen atom, and thus disfavor synthesis of the 1S,2R enantiomer.

In contrast, the orientation shown in FIGURE 5A results from an approach from the front, i.e. the side where the R1 phenyl group 21 is below the plane of the catalyst, and thus not in the way. For this reason, the approach depicted in FIGURE 5A is sterically favored, and thus synthesis of the 1R,25 enantiomer is favored.

It should be borne in mind that, although the above-described mechanism accurately predicts the high degree of enantioselectivity observed in the catalysts of the present invention, the mechanism is at present only a theory. As such, the proposed mechanism should in no way limit the scope of the present invention as defined by the appended claims.

It is noted that synthesis of the 1S,2R enantiomer of the cis-β-methyl styreneoxide is favored by using the S,S enantiomer of the catalyst.

It is also noted that this most preferred catalyst has C2 symmetry, i.e. it is identical when rotated 180 degrees. Consequently, whether the oxygen atom is aligned on the top of the catalyst as shown, or the bottom of the catalyst, the result is exactly the same.

In alternative embodiments, the catalyst has only approximate C₂ symmetry. In particular, as per the rules described above, the groups are positioned on R1-R4 so that when rotated 180°, the blocking groups are in the same place and the non-blocking groups are in the same place. Consequently, the enantioselectivity of the catalyst is maintained because the oxygen can be complexed to either side of the catalyst while achieving roughly the same steric hindrances which favor the approach of the prochiral olefin from one side.

In other alternative embodiments, the catalyst has only one non-blocking group. As a result, there is a favored approach only when the oxygen is aligned on one side of the catalyst. Thus, the enantioselectivity of the catalyst is maintained.

### The Second Aspect of the Invention

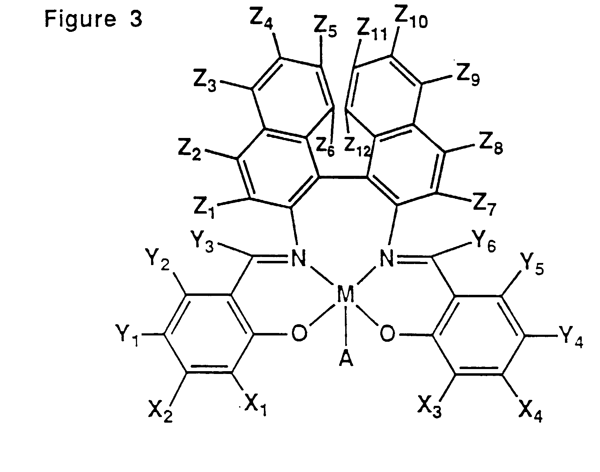

In accordance with the second aspect of the present invention, the chiral catalyst is made with a binapthyl diamine and has the following general formula (II) (see also FIGURE 3):

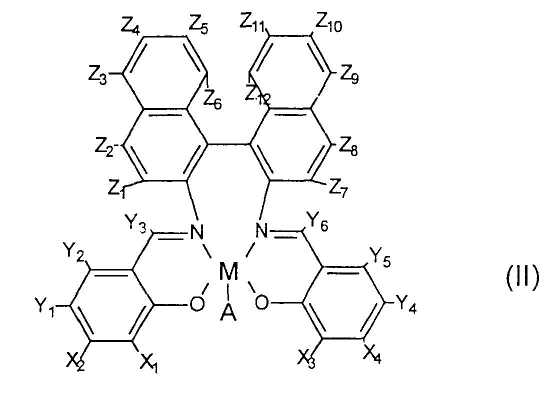

In this binapthyl embodiment, the transition metal ion M and the anion A are preferably selected from the same group as that discussed above with FIGURE 1. Also as above, it is required that at least one of X1 and X2 together with at least one of X3 and X4 are occupied by a group selected from the group of blocking substituents consisting of primary secondary or tertiary alkyl groups, aryl groups, hetero atoms and alkyl groups bearing hetero-atom substituents such as alkoxy or halide. Preferably, it is the X1 and X3 sites which bear one of these substituents. More preferably, X1 and X3 bear the same substituent, which substituent is most preferably a tertiary alkyl group, such as tertiary butyl.

The substituents on the Y3 and Y6 sites affect the conformation of the ligand and thus have an influence on enantioselectivity in the epoxidation. Preferably, Y3 and Y6 are hydrogen, methyl, alkyl, or aryl. More preferably, they are hydrogen or methyl. Most preferably, they are hydrogen.

The substituents Z1 and Z2 affect the differentiation between the faces of the proposed metal oxo and thus have an influence on enantioselectivity in the epoxidation. Preferably, Z1 and Z2 are hydrogen, ethyl, alkyl, silyl, or aryl. More preferably, they are alkyl or aryl groups.

The Y1, Y2, Y4, and Y5 sites on the catalyst of this second aspect are also seen to be less critical. As above, these sites are preferably occupied by hydrogen, although these sites may also be occupied by substituents independently selected from the group consisting of hydrogen, halides, alkyls, aryls, alkoxy groups, nitro groups.

This binapthyl alternative embodiment effects the same enantioselectivity as that of the preferred catalysts shown in the other figures. In particular, the configuration of the binaphthyl ligand provides for one of the napthyl groups to be above the plane of the catalyst and the other naphthyl group to be below the plane of the catalyst, thereby favoring approach to the oxygen atom from one side.

### The Third Aspect of the Invention

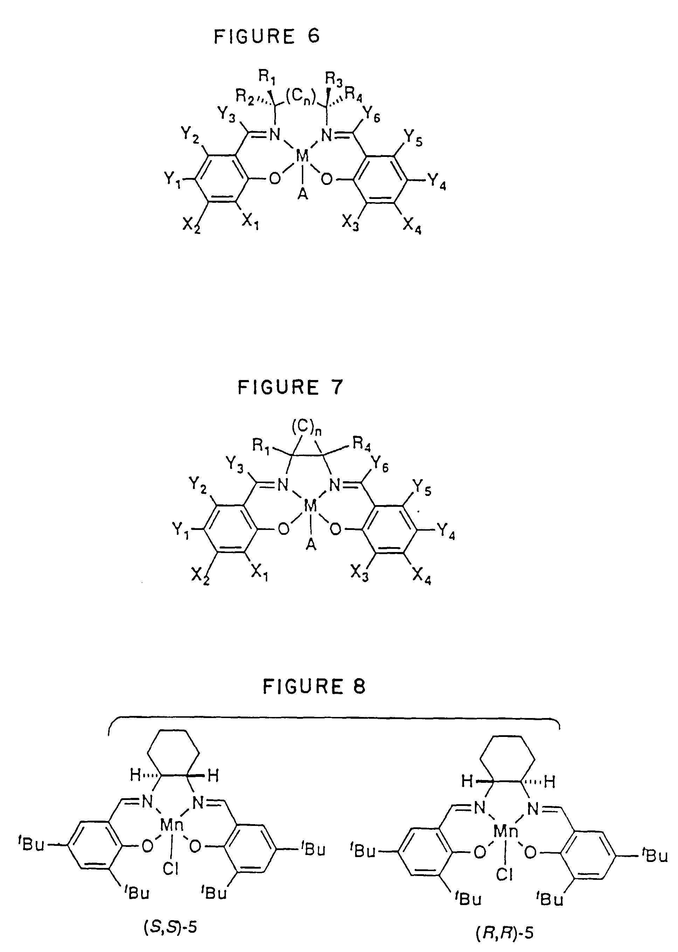

FIGURE 6 shows the structure of the third aspect of the present invention. In accordance with this aspect, the chiral catalyst has the following general formula (III):

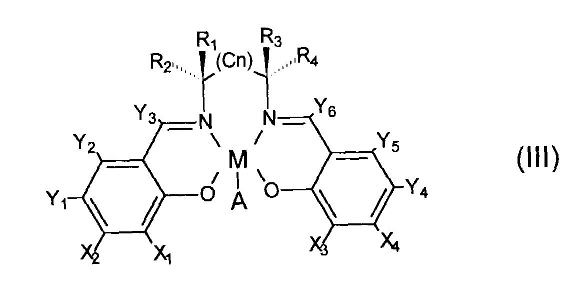

As with the first and second aspects, M is a transition metal ion selected from the group mentioned above, with Mn being the most preferred.

Likewise, A is an anion selected from the group mentioned above, with Cl being most preferred.

Also, n can be either 0, 1, or 2, with 0 being the most preferred.

As with the first and second aspects, there is a blocking substituent on either X1, X2 or both. This blocking substituent is selected from the group consisting of aryls, primary alkyls, secondary alkyls, tertiary alkyls, and hetero atoms. There is also a blocking substituent selected from the same group on either X3, X4 or both. Preferably, the blocking substituent are at X1 and X3, more preferably they are the same group, most preferably tert-butyl.

As a point of difference with the first aspect, the third aspect requires a blocking substituent located at at least one of Y1 and Y2, and at least one of Y4 and Y5. These blocking substituents are selected from the group consisting of aryls, primary alkyls, secondary alkyls, tertiary alkyls, and hetero atoms. The importance of these "side" blocking substituents will be discussed below.

In this third aspect, substituents Y3 and Y6 are independently selected from the group consisting of hydrogen and primary alkyl groups. Preferably, Y3 and Y6 are hydrogen.

Also in this third aspect, at least one of R1, R2, R3 and R4 is hydrogen. Where R1 is hydrogen, R3 is a primary alkyl. Where R2 is hydrogen, R4 is a primary alkyl. Where R3 is hydrogen, R1 is a primary alkyl. Finally, where R4 is hydrogen, R2 is a primary alkyl. Preferably, R1 and R4 are both hydrogen and R2 and R3 are a primary alkyl. Most preferably, R2 and R3 are methyl.

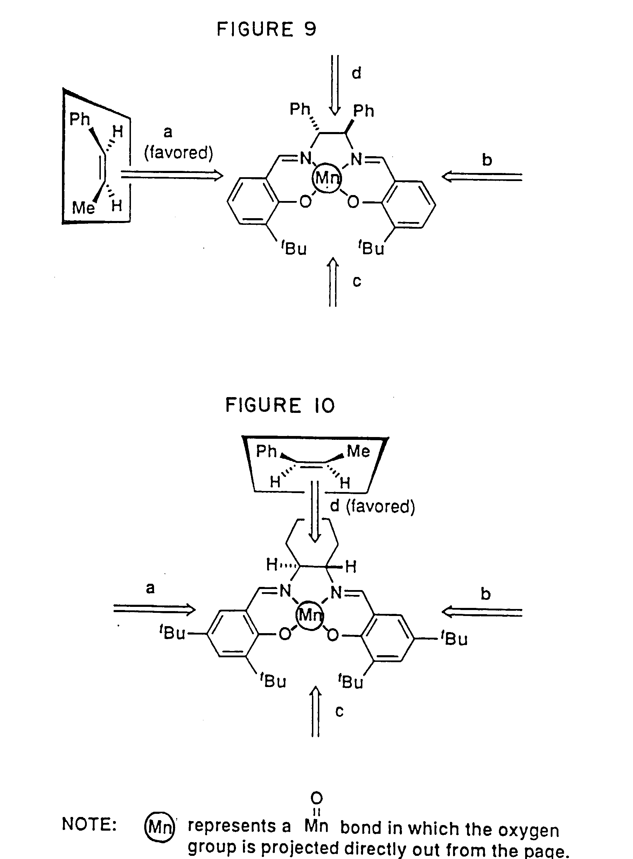

As can be seen, the catalyst of this third aspect is similar to the catalyst of the first aspect with the exception that the third aspect requires blocking substituents at the side positions of the catalyst, i.e. on the Y1 and/or Y2, and Y4 and/or Y5 sites. Also, either one or two of R1, R2, R3 and R4 is required to be an hydrogen, with the remaining substituents at the R sites required to be primary alkyls in the defined arrangement. The importance of this configuration and the proposed mechanism for the catalyst of the third aspect is discussed below in connection with FIGURE 10.

### The Fourth Aspect of the Invention

FIGURE 7 shows the structure of a catalyst of the fourth aspect of the invention. This catalyst has the general formula (IV):

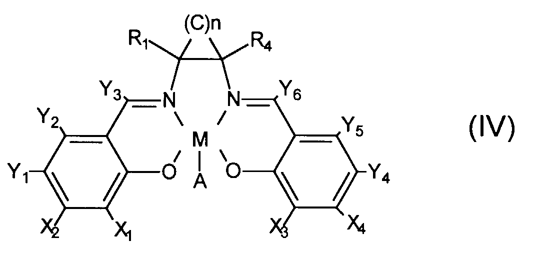

In this embodiment, the transition metal, M, and the anion, A, are selected from the same groups as above with the same preferences.

Likewise, the substituents at X1, X2, X3, X4, Y1, Y2, Y4, and Y5 are selected from the same groups as in the third aspect described above with the same preferences. In other words, this embodiments requires blocking substituents at the "bottom" and "sides" as does the third aspect. Most preferably, X1, X3, Y1, and Y4 are all t-butyl.

The requirements and preferences for Y3 and Y6 are the same as with the third aspect. Preferably, Y3 and Y6 are hydrogen.

As can be seen, this catalyst of the fourth aspect of the invention includes a ring attached to the two nitrogen atoms, which ring is n+2 carbons long. In this catalyst, n can be 3, 4, 5 or 6. Preferably n is 4. The carbons in the Cₙ portion can have substituents selected from hydrogen, alkyl, aryl, and hetero atoms. Preferably, the substituents on the carbons in the Cₙ portion are hydrogen.

In this fourth aspect, R1 and R4 are configured so as to be trans to each other. Also, R1 and R4 are selected from the group consisting of primary alkyls and hydrogen. Preferably, R1 and R4 are the same. Most preferably, both are R1 and R4 are hydrogen.

Conceptually, the carbons in the ring which are adjacent the carbons which in turn are adjacent the nitrogen atoms are attached to what were shown as the R2 and R3 sites in the third aspect (FIGURE 6). Thus, this fourth aspect is, in some respects, a subset of the third aspect with the two ends of the n carbon chain (a primary alkyl) being attached to the R2 and R3 sites.

One distinction between the third and fourth aspect is that the catalyst of the fourth aspect has R1 and R4 which can be either hydrogen or a primary alkyl.

FIGURE 8 shows a preferred catalyst made according to this fourth aspect of the invention. As can be seen, in this embodiment, the ring is a six-membered ring, that is, n=4. Also, R1 and R4, which are trans to each other, are hydrogen. X1, X3, Y1, and Y4 are all t-butyl. All other substituents are hydrogen.

FIGURES 9 and 10 illustrate the distinction between the mechanism proposed for the first and second aspects of the invention and the proposed mechanism for the third and fourth aspects.

FIGURE 9, representing the first and second aspects of the invention, shows the proposed favored approach of the prochiral olefin. Approach c is believed to be disfavored by the bulky t-butyl groups. Approach d is similarly unfavorable, due to the steric bulk of the phenyl groups on the catalyst. Approaches a and b are differentiated by the dissymmetry of the catalyst. As shown in the depicted embodiment, because the phenyl to the left is below the page and the phenyl to the right is above the page, it is predicted that approach b will be less favorable due to steric interactions between the olefin and the phenyl group. In the context of the more favored approach from the left (approach a), it is predicted that the more favorable approach of the olefin to the oxo group is such that the larger substituent on the olefin is oriented away from the t-butyl groups on the catalyst.

FIGURE 10, representing the third and fourth aspects of the invention, shows the proposed favored approach of the olefin when the catalyst has side blocking groups. It is believed that, because of the side t-butyl groups at Y1 and Y4, the approaches **a** from the left and **b** from the right are disfavored. Likewise, because of the bottom blocking groups at X1 and X3, the approach **c** from the bottom is also disfavored. Thus, approach **d** from the top is favored. In addition, because of the chirality of the catalyst, the orientation of the prochiral olefin is also influenced. As shown in this depicted embodiment, because of greater steric hindrance on right, the olefin is predicted to orient itself with the larger group on the left.

Because approach **d** is theorized to be the favored approach, the groups at R1 and R4 are limited to hydrogen and primary alkyls. In other words, it is believed that larger groups would block the approach d.

It should be noted that, although the above discussion is consistent with the observed results, the proposed mechanism for all four aspects of the invention is only theorized at this point. Consequently, the explanation is not to be viewed as limiting the scope of the invention as defined in the appended claims.

The preferred route to prepare the chiral catalysts of the present invention is a condensation reaction with the substituted salicylaldehyde and the substituted diamine. In general, quantities of these compounds are reacted in a 2 to 1 molar ratio in absolute ethanol. The solutions are refluxed typically for 1 hour, and the salen ligand is either precipitated in analytically pure form by addition of water, or the metal complex is generated directly by addition of the metal as its acetate, halide, or triflate salt.

The following procedure is general for the preparation of:

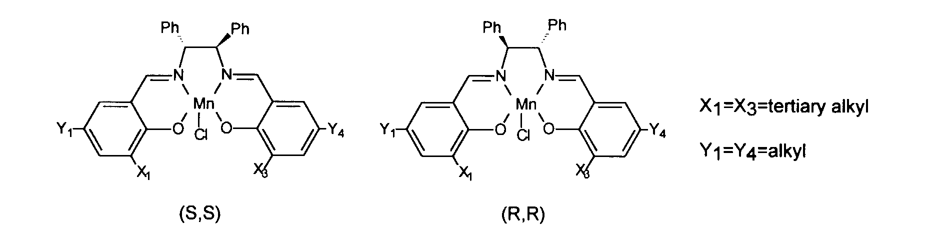

The salen ligand is redissolved in hot absolute ethanol to give a 0.1 M solution. Solid Mn(OAC)₂·4H₂O (2.0 equivalents) is added in one portion and the solution is refluxed for 1 h. Approximately 3 equivalents of solid LiCl are then added and the mixture is heated to reflux for an additional 0.5 h. Cooling the mixture to 0°C affords the Mn(III) complex 1 as dark brown crystals which are washed thoroughly with H₂O and isolated by filtration in ≈75% yield. An additional crop of material can be obtained by dropwise addition of H₂O to the mother liquor. Combined yields of catalyst are 89-96% for this step, and 81-93% overall from the optically pure 1,2-diphenylethylene diamine. Acceptable C, H, N, Cl, and Mn analyses of each of the catalysts have been obtained (±0.4%), although these vary according to the extent of water and ethanol incorporation in the powdery product. Enantioselectivities in the epoxidation reactions are invariant with different batches of a given catalyst, indicating that the solvent content of the catalysts does not influence its effectiveness.

Another example of the method of preparing the catalyst is described as follows: Most preferably, the starting diamine is R,R- or S,S-1,2-diamino-1,2-diphenylethane and the starting salicylaldehyde is 3-tert-butylsalicylaldehyde.

A solution of 2.0 mmol of 3-tert-butylsalicylaldehyde in 3 ml of absolute ethanol is added dropwise to a solution of 1.0 mmol of (R,R)-1,2-diamino-1,2-diphenylethane in 5 ml of ethanol. The reaction mixture is heated to reflux for 1 h and then 1.0 mmol of Mn(OAc)₂ 4H₂O is added in one portion to the hot (60°C) solution. The color of the solution immediately turns from yellow to brown upon addition. It is refluxed for an additional 30 min and then cooled to room temperature. A solution of 10% NaCl (5ml) is then added dropwise and the mixture stirred for 0.5h. The solvents are then removed in vacuo and the residue is triturated with 50 ml of CH₂Cl₂ and 50 ml of H₂O. The organic layer is separated and the brown solution was washed with saturated NaCl. Separation of the organic phase and removal of solvent resulted in a crude material which was recrystallized from C₆H₆/C₆H₁₄ to give 0.938 mmol of 1 (93.8%).

In accordance with the epoxidation method aspect of the invention, the prochiral olefin, an oxygen atom source, and the chiral catalyst are reacted under such conditions and for such time as is needed to epoxidize said olefin.

The prochiral olefin can be selected from mono-substituted, 1,1-disubstituted, cis-1,2-disubstituted, trans-1,2-disubstituted, trisubstituted, and tetrasubstituted olefins. Of these, the monosubstituted and cis-1,2-disubstituted olefins have shown the highest ee values.

Preferably, the prochiral olefin to be epoxidized is selected from the group consisting of cis-disubstituted olefins, including cyclic olefins, bearing a sterically demanding substituent on one end and a smaller substituent on the other end. More preferably, the prochiral olefin is a cis disubstituted olefin with a primary substituent on one side of the double bond and a secondary, tertiary, or aryl substituent on the other side.

The prochiral olefin can also be selected from the group consisting of enamines, enols, and alpha, beta-unsaturated carbonyl compounds. More preferably, the prochiral olefin is selected from the group consisting of cis-β-methylstyrene, dihydronaphthalene, 2-cyclohexenyl-1,1-dioxolane, propylene, styrene and 2,2-dimethylchromene. Most preferably, the prochiral olefin is cis-β-methylstyrene.

The oxygen atom source used in the epoxidation reaction should be an oxidant which is relatively unreactive toward olefins under mild conditions. Preferably, the oxygen atom source is selected from the group consisting of NaOCl, iodosylmesitylene, NaIO₄, NBu4I04, potassium peroxymonosulfate, magnesium monoperoxyphthalate, and hexacyanoferrate ions. More preferably, the oxygen atom source is selected from the group consisting of NaOCl and iodosomesitylene. For economic reasons, the most preferred oxygen atom source is NaOCl.

The most preferred method uses NaOCl as the oxygen atom source. For convenience this method will be designated METHOD A. The most preferred details of METHOD A are as follows:

A solution of 0.05 M Na₂B₄O₇ 10H₂O (1.0ml) is added to a 2.5 ml solution of undiluted commercial household bleach (Chlorox®). The pH of the resulting buffered solution is approximately 9.5, and it is adjusted to a pH of about 10.5 by addition of a few drops of 1 M NaOH solution. To this solution is added a solution of 0.02 mmol of the preferred catalyst and 1.0 mmol of cis-β-methylstyrene in 2.0 ml of CH₂Cl₂. The twophase mixture is stirred at room temperature and the reaction progress is monitored by capillary gas chromatography. After approximately 3 hours, 10 ml of CH₂Cl₂ is added to the mixture and the brown organic phase is separated, washed twice with 10 ml H₂O and once with 10 ml saturated NaCl solution, and then dried for 15 minutes over anhydrous Na₂SO₄. The solution is filtered and solvent is removed under vacuum. The residue is purified by flash chromatography on silica gel using a 20:80 mixture of CH₂Cl₂:hexane as the eluting solvent. Pure epoxide is isolated as a colorless liquid in 70% yield (0.70 mmol) by combination of the product-containing fractions and removal of solvent under vacuum. The optical purity of this material is determined to be 85% ee by the method described below.

In a slightly less preferred embodiment, iodosylmesitylene is used as the oxygen atom source. For convenience, this method is designated as METHOD B and has the following preferred details: A solution of 1.0 mmol of olefin, 8 ml CH₂Cl₂ and 0.04 mmol of the catalyst are stirred at room temperature as solid iodosomesitylene is added in 0.3 mmol portions at 15-30 minute intervals. Disappearance of starting olefin is complete after addition of 6 portions (1.8 mmol) of total iodosylmesitylene. Solvent is removed in vacuo, the residue is extracted with hexane, and the mixture was filtered through Celite® to remove catalyst and other solids. Pure epoxide was obtained by flash chromatography (10g SiO₂, CH₂Cl₂/hexane 20:80 eluent). Enantiomeric excesses are determined by 1H NMR using Eu(hfc)₃ as a chiral shift reagent, or in the case of stilbene oxide by direct separation by HPLC on a commercial (Regis) covalently-bound leucine Pirkle column. Absolute configurations were assigned by comparison of aD with accepted literature values.

### EXAMPLES

The following examples are provided by way of explanation and illustration. As such, these examples are not to be viewed as limiting.

### Preparation of the Catalysts

### Procedures for the Preparation of Chiral Salen Based Catalysts

Preparation of:

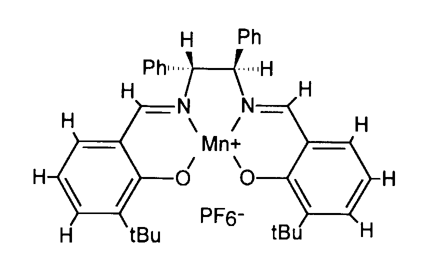

### (R,R)-1,2-Diphenyl-1,2-bis (3-ter-butylsalicylideamino)-ethane (2).

A solution of 360.5 mg (2.0mmol) of 3-tert.-butylsalicylaldehyde in 3 ml of EtOH was added dropwise to a solution of 212.3 mg (1.0 mmol) of (R,R)-1,2-diamino-1,2-diphenylethane in 5 ml of EtOH. The reaction mixture was heated to reflux for 1 h and water (5 ml) was added. The oil that separated solidified upon standing. Recrystallization from MeOH/H₂O gave 485.8 mg (91%) of yellow powder, mp 73-4°C. 1H NMR (CDCl₃) δ 1.42 (s, 18H, CH₃), 4.72 (s,2H, CHN=C), 6.67-7.27 (m, 16H, ArH), 8.35 (s, 2H, CH=N), 13.79 (s, 2H, ArOH) ppm; 13C NMR (CDCl₃) δ 29.3, 34.8, 80.1, 117.8, 118.5, 127.5, 128.0, 128.3, 129.6 130.1, 137.1, 139.5, 160.2, 166.8 ppm. Anal. Calcd. for. C₃₆H₄₀N₂O₂. C, 81.17;H, 7.57; N, 5.26. Found: C,81.17; H, 7.60; N, 5.25.

### ((R,R)-1,2-Diphenyl-1,2-bis(3-tert-butylsalicylideamino)ethane)-manganese(II) Complex (3).

Under strictly air-free conditions, a solution of 64.0 mg (1.6 mmol) of NaOH in 2 ml of MeOH was added dropwise to a solution of 426. 1 mg (0.8 mmol) of **(2)** in 5 ml of EtOH with stirring under an atmosphere of nitrogen. A solution of 196.1 mg (0.8 mmol) of Mn(OAc)₂-4H₂O in 3 ml of MeOH was added rapidly and the orange mixture was stirred for 24 hr. The solvent was removed in vacuo and the residue was stirred with 5 ml of benzene and filtered to remove NaOAe. The filtrate was concentrated to about 1 ml and 3 ml of hexane was added. The mixture was cooled to -30°C and the precipitate was collected by filtration to give 410.2 mg (87%) of orange powder. Anal. Calcd. for C₃₆H₃₈MnN₂O₂-(CH₃OH)0.5; C,72.86; H, 6.70; N, 4.66. Found: C, 73.05; H, 6.76; N,4.39.

### ((R,R) -1,2-Diphenyl-1,2-bis (3-tert-butylsalicylideamino) ethane) -manganese (III) hexafluorophosphate ((R,R)-1).

A solution of 165.5 mg (0.5 mmol) of ferrocenium hexafluorophosphate in 2 ml of CH₃CN was added dropwise to a solution of 292.8 mg (0.5 mmol) of **(3)** in 3 ml of CH₃CN under N₂. The reaction mixture was stirred for 30 min and the solvent was removed in vacuo. The residue was triturated with 5 ml of hexane and filtered. The solid was then washed with hexane until the filtrate was colorless and dried under vacuum to give 360.5 mg (93%) of 1 as a brown powder. IR CH₂Cl₂) 2955, 1611, 1593, 1545, 1416, 1389, 1198, 841 cm-1. Anal. Calcd. for C₃₆H₃₈F₆MnN₂O₂P·(H₂O)1.5· (CH₃CN)0.5: C, 56.93; II, 5.30; N, 4.57. Found; C, 57.11; H, 5.50; N, 4.50.

**Preparation of:**

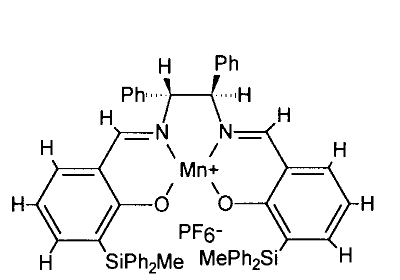

The salicylaldedyde derivative **(4)** was prepared by the following sequence using well-established procedures in each step:

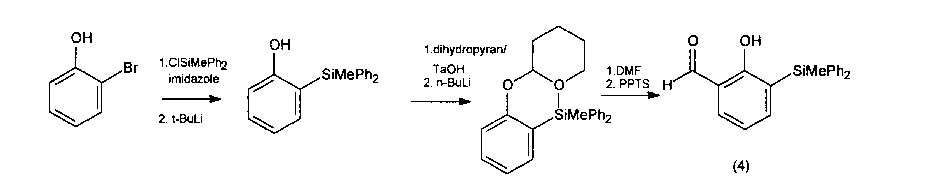

### (R,R) -1,2-Diphenyl-1,2-bis (3-diphenylmetlylsilylsalicylideamino)ethane (5).

A solution of 348.3 mg (1.09 mmol) of **(4)** and 116.0 mg (0.546 mmol) of (R,R)-1,2-diamino-1,2-diphenylethane in 5 ml of ethanol was heated to reflux for 0.5 h. A bright yellow oil separated from the solution and it solidified upon standing. The mixture was filtered and the yellow solid was washed with 2 x 5 ml ethanol. The isolated yield of product pure by 1HNMR analysis was 416 mg (97%). 1HNMR (CDCl₃) 80.95 (s, 3H), 4.68 (s, 2H), 6.72-7.55 (m, 36H, ArH), 8.37 (s,WH), 13.34 (s, 2H) ppm.

### ((R,R) -1,2-Diphenyl-1,2-bis (3-diphenylmetlylsilylsalicylideamino)ethane)-manganese(II) Complex (6).

Under strictly air-free conditions, a solution of 32.0 mg (0.48 mmol) of KOH in 2 ml of ethanol was added dropwise to a suspension of 195 mg (0.24 mmol) of **(5)** in 3 ml of ethanol with stirring. The heterogeneous mixture was stirred for 20 min, and solution of 51.5 mg (0.24 mmol) of Mn(OAc)₂·4H₂O in 3 ml of McOH was then added rapidly and the yellow-orange mixture was stirred for 8 hr. at room temperature then refluxed under N₂ for 4 hr. The solvent was removed in vacuo and the residue was washed 5 ml of methanol, 5 ml of ethanol, and isolated by filtration. Yield of orange product was 188 mg (90%). This material was used in the next step without any further purification or analysis.

### ((R,R) -1,2-Diphenyl-1,2-bis (3-diphenylmetlylsilylsalic-ylideamino)ethane) -manganese(III) hexafluorophosphate ((R,R)-1.

A solution of 72 mg (0.217 mmol) of ferrocenium hexafluorophosphate in 2 ml of CH₃CN was added dropwise to a solution of 188 mg (0.217 mml) of **(6)** in 3 ml of CH₃CN under N₂. The reaction mixture was stirred for 30 min and the solvent was removed in vacuo. The solid residue was then washed with hexane until the filtrate was colorless. The brown powder was dried under vacuum to give 201.3 mg (92%) of 7. Anal. Calcd. for C₅₄H₄₆F₆MnN₂O₂PSi₂· (CH₃CN) 1.5 (H₂O); C, 62.77; II, 4.85; N, 4.50. Found: C, 62.89; H, 4.47; N, 4.57.

**Preparation of:**

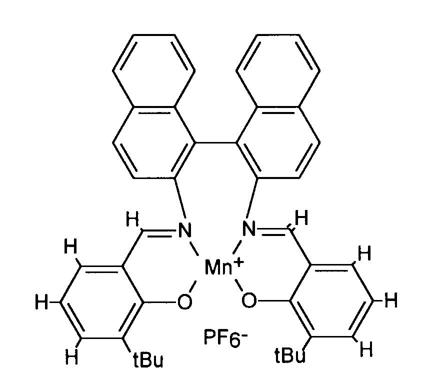

### 2,2'-Bis (3-tert-Butylsalicylideamino)-1,1'-binaphthyl.

A solution of 725 mg (4.0mmol) of 3-tert-butyl-salicylaldehyde in 6 ml of EtOH was added dropwise to a solution of 569 mg (2.0 mmol) of (+)-2,2'diamino-1,1-binaphthyl in 5 ml of EtOH. The reaction mixture was heated to reflux for 8 h and then volatile materials were removed under vacuum. The residue was purified by flash chromatography on 80 g SiO2, using 20% CH₂Cl₂ in hexane as eluent. The mobile yellow fraction was collected and solvents were removed under vacuum to give 725 mg (1.20 mmol, 59% yield) of the diimine as a yellow powder.

### 1,1'-Binaphthyl-2,2'-bis (3-tert-butylsalicylideamino)--manganese(II) Complex.

Under strictly air-free conditions, a solution of 2 mmol of KOH in 2 ml of MeOH is added dropwise to a solution of 1 mmol of 2,2'-bis(3-tert-butylsalicylideamino)-1,1'-binaphthyl in 5 ML of EtOH with stirring under an atmosphere of nitrogen. A solution of 1 mmol of Mn(OAc)₂·4H₂O in 3 ml of McOH is added rapidly and the orange mixture is stirred for 24 hr. The solvent is removed in vacuo and the residue was stirred with 5 ml of benzene and filtered to remove KOAc. The filtrate is concentrated to dryness to afford the Mn(II) complex as an orange powder.

### 1,1'-Binaphthyl-2,2'-bis (3-tert-butylsalicylideamino)--manganese(III) hexafluorophosphate.

A solution of 165.5 mg (0.5 mmol) of ferrocenium hexafluorophosphate in 2 ml of CH₃CN is added dropwise to a solution of 0.5 mmol of 1,1'-Binaphthyl-2,2' bis(3-tert Butylsalicylideamino)-manganese(II) Complex in 3 ml in vacuo. The residue is triturated with 5 ml of hexane and filtered. The solid is then washed with hexane until the filtrate is colorless and dried under vacuum to give the Mn(III) salt as a deep green powder.

**(d) Preparation of:**

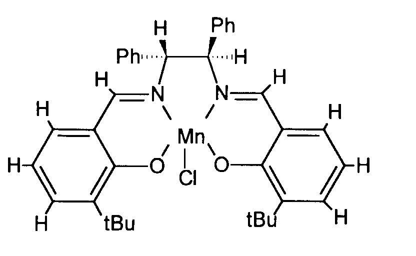

No precautions to exclude air or moisture were necessary in this procedure. A solution of 360.5 mg (2.0 mmol) of 3-tert butylsalicylaldehyde in 3 ml of absolute ethanol was added dropwise to a solution of 212.3 mg (1.0 mmol) of (R,R)-1,2-diamino 1,2-diphenylethane in 5 ml of ethanol. The reaction mixture was heated to reflux for 1 h and then 245.1 mg (1.0 mmol) of Mn(OAc)₂·4H₂O was added in one portion to the hot (60°C) solution. The color of the solution immediately turned from yellow to brown upon addition. It was refluxed for an additional 30 min and then cooled to room temperature. A solution of 10% NaCl (5 ml) was then added dropwise and the mixture stirred for 0.5 h. The solvent was then removed in vacuo and the residue was triturated with 50 ml of CH₂Cl₂ and 50 ml of H₂O. The organic layer is separated and the brown solution was washed with saturated NaCl. Separation of the organic phase and removal of solvent afforded crude material which was recrystallized from C₆H₆/C₆H₁₄ to give 591 mg (0.938 mmol) of the chloride salt of 1 (94%). Anal. Calcd. for C₃₆H₃₈ClMnN₂O₂·(H₂O)0.5: C, 68.63; H, 6.24; N, 4.45. Found: C, 69.01; H, 6.26; N, 4.38.

### Procedure for the preparation of the most preferred catalyst of the fourth aspect

### (R,R)- and (S,S)-1,2,-bis(3,5-di-tert-butylsalicylide-amino) cyclohexane

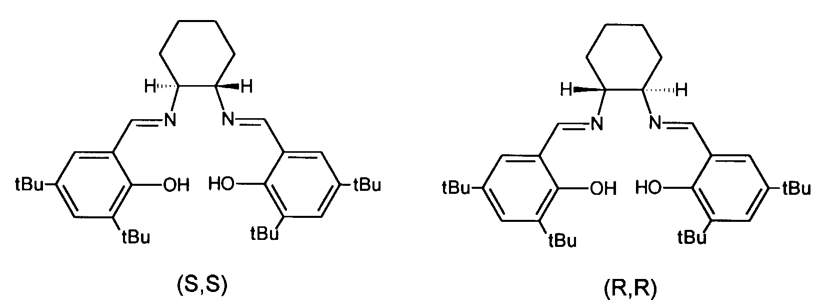

3,5-Di-t-butylsalicylaldehyde (2.0 equivalents) was added as a solid to a 0.2 M solution of (R,R) or (S,S) 1,2 diaminocyclohexane (1.0 equivalent) in absolute ethanol. The mixture was heated to reflux for 1 hr. and then H₂O was added dropwise to the cooled bright yellow solution. The resulting yellow crystalline solid was collected by filtration and washed with a small portion of 95% ethanol. The yield of analytically pure salen ligand obtained in this manner was 90-97%. Spectroscopic and analytical data for the salen ligand: ¹H NMR (CDCl₃) δ 13.72 (s, 1 H), 8.30 (S, 1H), 7.30 (d, J = 2.3 Hz, 1H), 6.98 (d, J = 2.3 Hz, 1H), 3.32 (m, 1H), 2.0-1.8 (m, 2H), 1.8-1.65 (m, 1H), 1.45 (m, 1H), 1.41 (s, 9H), 1.24 (s, 9H). ¹³C NMR (CDCl₃): δ 165.8,, 158.0, 139.8, 136.3, 126.0, 117.8, 72.4, 34.9, 33.0, 31.4, 29.4, 24.3. Anal. Calcd for C₃₆H₅₄N₂O₂: C, 79.07; H, 9.95; N, 5.12. Found: C, 79.12; H, 9.97; N, 5.12.

### (R,R)- and (S,S)-[1,2-bis(3,5-di-tert-butylsalicylideamino) cyclohexane] -manganese (III) chloride.

The salen ligand immediately above is redissolved in hot absolute ethanol to give a 0.1 M solution. Solid Mn(OAC)₂·4H₂O(2.5 equivalents) is added in one portion and the solution is refluxed for 1 hr. Approximately 5 equivalents of solid LiCl are then added and the mixture is heated to reflux for an additional 0.5 hr. Cooling the mixture to 0°C and addition of a volume of water equal to the volume of the brown ethanolic solution to afford the Mn(III) complex as a dark brown powder which are washed thoroughly with H₂O, and isolated by filtration in 81-93% yield. Acceptable C, H, N, Cl, and Mn analyses of the catalyst have been obtained (±0.4%), but these vary according to the extent of water and ethanol incorporation in the powdery product. Enantioselectivities in the epoxidation reactions are invariant with different batches of a given catalyst, indicating that the solvent content of the catalyst does not influence its effectiveness.

Analytical data for this catalyst: Anal. Calculated for C₃₆H₅₂ClMnN₂O₂·C₂H₅OH: C 67.19; H 8.31; Cl,5.22; Mn 8.09; N 4.12; Observed C 67.05; H 8.34; Cl 5.48; Mn 8.31; N 4.28.

### Procedures for the Asymmetric Epoxidation of Olefins

### Method A (NaOCl as oxygen atom source):

A solution of 0.05 M Na₂B₄O₇·10H₂O (1.0 ml) is added to a 2.5 ml solution of undiluted commercial household bleach (Chlorox®). The pH of the resulting buffered solution is approximately 9.5, and it is adjusted to a pH of 10.5 by addition of a few drops of 1 M NaOH solution. To this solution is added a solution of 0.005 to 0.02 mmol of the catalyst and 1.0 mmol of olefin in 2.0 ml of CH₂Cl_{2.} The two-phase mixture is stirred at room temperature and the reaction progress is monitored by capillary gas chromatography. Reactions are complete within approximately 1-5 hours. After progress of the reaction is complete, 10 ml of CH₂Cl₂ is added to the mixture and the brown organic phase is separated, washed twice with 10 ml H₂O and once with 10 ml saturated NaCl solution, and then dried for 15 min over anhydrous Na₂SO₄. The solution is filtered and solvent is removed under vacuum. The residue is purified by standard procedures using flash chromatography on 10g of silica gel using a mixture of CH2Cl2/hexane as the eluting solvent. Pure epoxide is isolated by combination of the product-containing fractions and removal of solvent under vacuum. The optical purity of this material is determined by the method described below.

### Method B (iodosylmesitylene as oxygen atom source)

A solution of 1.0 mmol of olefin, 8 ml CH₂Cl₂ and 0.04-0.08 mmol of the catalyst is stirred at room temperature as solid iodosomesitylene is added in 0.3 mmol portions at 15-30 minute intervals. Disappearance of starting olefin is complete after addition of 4 to 10 portions (1.2 to 3 equivalents) of total iodosylmesitylene. Solvent is removed in vacuo, the residue is extracted with hexane, and the mixture was filtered through Celite® to remove catalyst and other solids. Pure epoxide is obtained by flash chromatography (10g SiO₂, CH₂Cl₂/hexane eluent). Enantiomeric excesses are determined by 1H NMR using Eu(hfc)₃ as a chiral shift reagent, or in the case of stilbene oxide by direct separation by HPLC on a commercial (Regis) covalently-bound leucine Pirkle column. Absolute configurations were assigned by comparison of [α]D with accepted literature values.

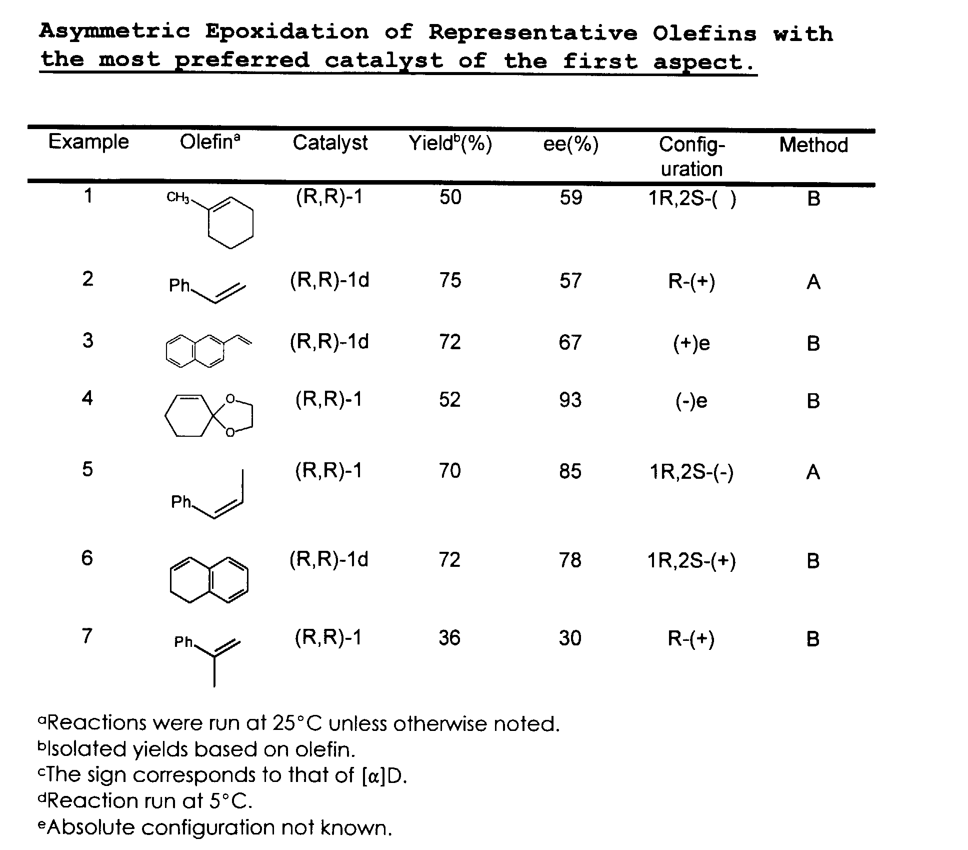

The table above shows that the best enantiomeric excess values were observed with Examples 4, 5, and 6, i.e. cis disubstituted olefins. In contrast, Example 7, a 1,1 disubstituted olefin, had the lowest ee values. Example 1, a trans disubstituted olefin, and Examples 2 and 3, monosubstituted olefins, had intermediate ee values.

### Asymmetric Epoxidation of Representative Olefins with Catalysts from the first and fourth aspects of the invention.

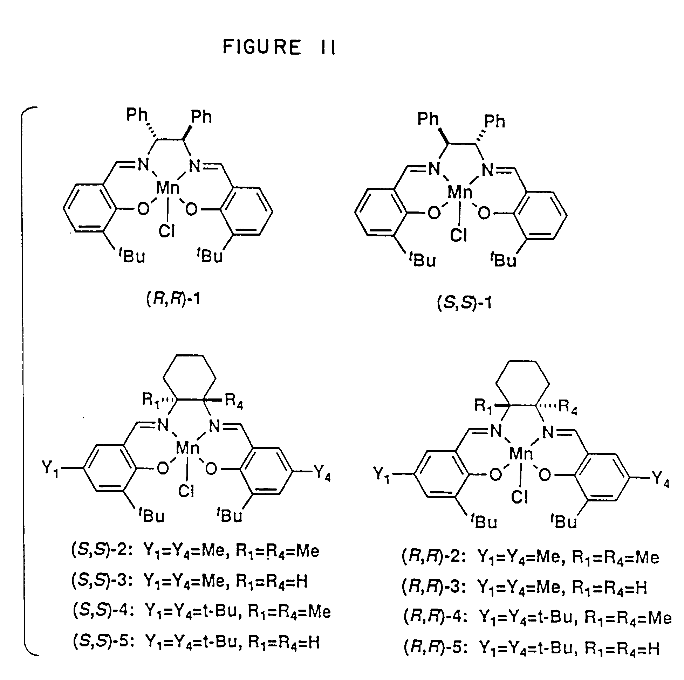

The following Examples 8-16 were run the same as Examples 1-7, except that different catalysts were used. The key to the catalyst numbering system is found in FIGURE 11. As can be seen, Example 8 was made according to the most preferred embodiment of the first aspect. Examples 9-16 were made according to the fourth aspect, with the catalyst used in Examples 12-16 being the most preferred embodiment of the fourth aspect. It is also noted that all of Examples 8-16 were run with method B described above.

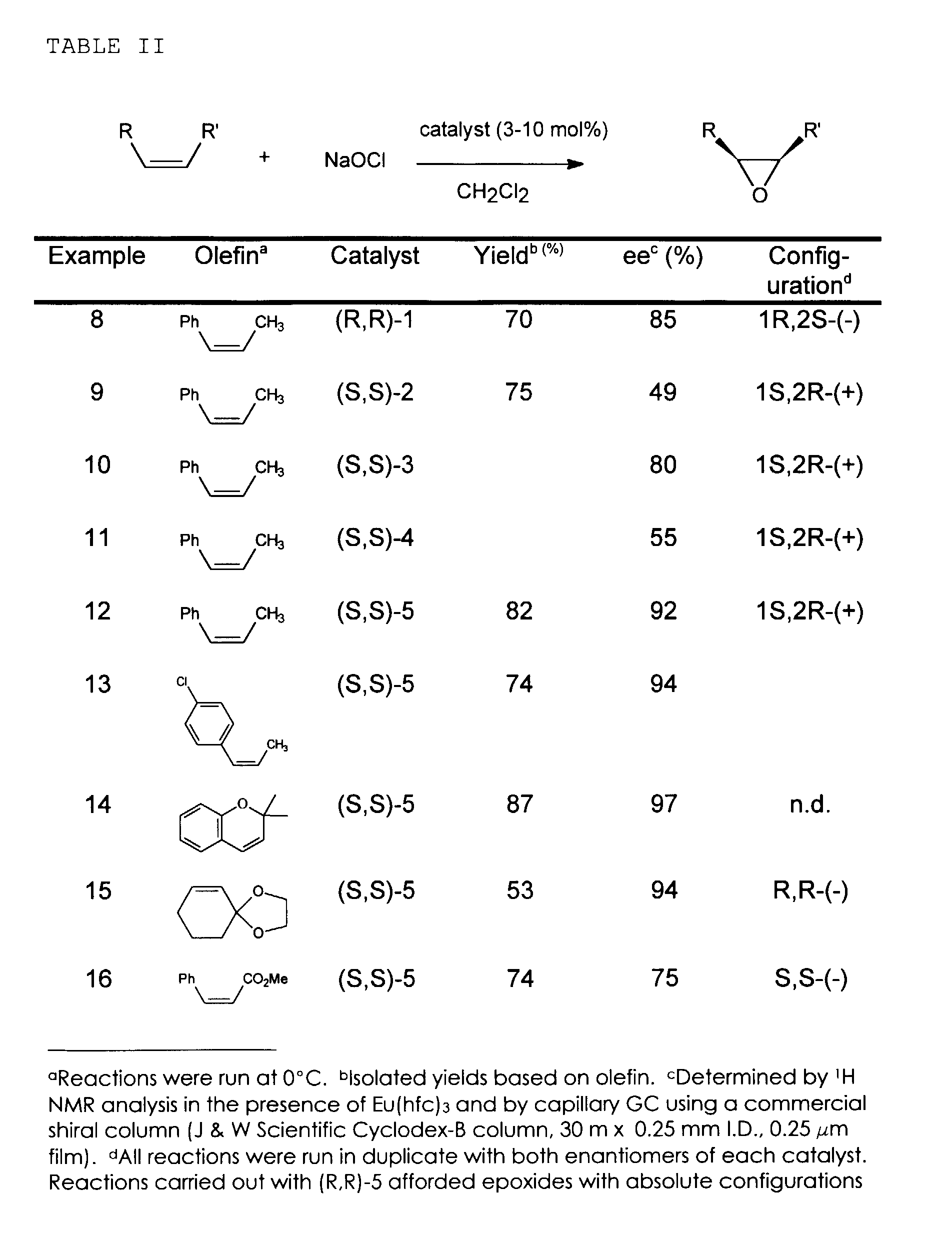

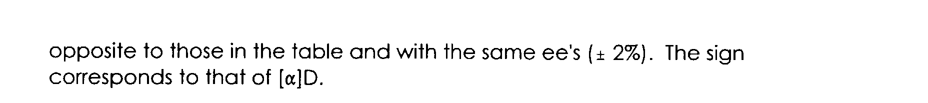

As shown in Examples 12-15, the most preferred catalyst of the fourth aspect catalyzes the epoxidation of cis-disubstituted olefins with excellent enantioselectiveness.

It should be noted that, although much of the discussion has involved the use of salen derivatives (made from ethylenediamines), salpn derivatives (made from propylenediamines) and salbn derivatives (made from butylenediamines) are also within the scope of the present invention.

## Claims

1. A chiral catalyst having the general formula (I): 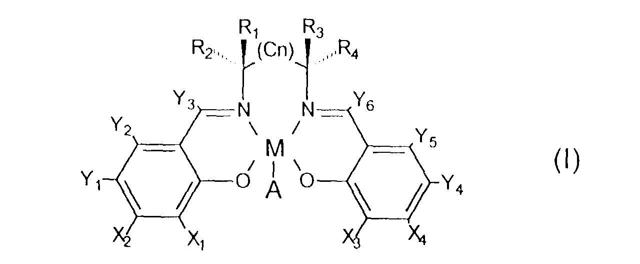
where M is a transition metal ion;
where A is an anion;
where n is either 0, 1, or 2;
where at least one of X1 or X2 is selected from the group consisting of aryls, primary alkyls, secondary alkyls, tertiary alkyls, hetero atoms, and alkyl groups bearing hetero atoms;
where at least one of X3 or X4 is selected from the group consisting of aryls, primary alkyls, secondary alkyls, tertiary alkyls, hetero atoms, and alkyl groups hearing hetero atoms;
where Y3 and Y6 are independently selected from the group consisting of hydrogen, halides, alkyls, aryls and alkyl groups hearing hetero atoms, and Y1, Y2, Y4 and Y5 are independently selected from the group consisting of hydrogen, halides, alkyls, aryls, alkyl groups bearing hetero atoms, alkoxy groups and nitro groups;
where at least one of R1, R2, R3 and R4 is selected from a first group consisting of hydrogen, CH₃, C₂H₅ and primary alkyls;
where, if R1 is selected from said first group, then R2 and R3 are selected from a second group consisting of aryls, secondary alkyls, tertiary alkyls, and alkyl groups bearing hetero atoms;
where, if R2 is selected from said first group, then R1 and R4 are selected from said second group;
where, if R3 is selected from said first group, then R1 and R4 are selected from said second group;
where, if R4 is selected from said first group, then R2 and R3 are selected from said second group; and where the carbon atoms in the (Cₙ) portion have substituents selected from the group consisting of hydrogen, alkyl, aryl and hetero atoms.

2. The catalyst of Claim 1 wherein the transition metal ion is selected from the group consisting of Mn, Cr, Fe, Ni, Co, Ti, V, Ru, and Os.

3. The catalyst of Claim 1 wherein the transition metal ion is selected from the group consisting of Mn, Cr, Fe, Ni, and Co.

4. The catalyst of Claim 1 wherein the metal ion is Mn.

5. The catalyst of Claim 1 wherein said first group consists of hydrogen and methyl.

6. The catalyst of Claim 1 wherein said first group consists of hydrogen.

7. The catalyst of Claim 1 wherein said second group consists of t-butyl and phenyl.

8. The catalyst of Claim 1 wherein said second group consists of phenyl.

9. The catalyst of Claim 1 wherein R1 is the same as R4 and R2 is the same as R3.

10. The catalyst of Claim 9 wherein said first group consists of hydrogen and methyl.

11. The catalyst of Claim 9 wherein said second group consists of t-butyl and phenyl.

12. The catalyst of Claim 1 wherein X1 and X3 are independently selected from the group consisting of t-butyl and phenyl.

13. The catalyst of Claim 12 wherein X1 and X3 are the same.

14. The catalyst of Claim 1 wherein both X1 and X3 are t-butyl.

15. The catalyst of Claim 9 wherein:
X1 and X3 are the same and are selected from the group consisting of aryls, primary alkyls, secondary alkyls, tertiary alkyls, hetero atoms, and alkyl groups bearing hetero atoms.

16. The catalyst of Claim 15 wherein the metal ion is manganese.

17. The catalyst of Claim 15 wherein said first group consists of hydrogen.

18. The catalyst of Claim 15 wherein said second group consists of t-butyl and phenyl.

19. The catalyst of Claim 15 wherein said second group consists of phenyl.

20. A chiral catalyst having the general formula (II) : 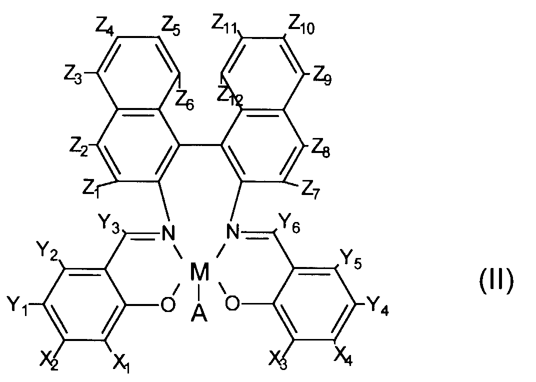 where M is a transition metal ion;
where A is an anion;
where at least one of X1 or X2 is selected from the group consisting of aryls, primary alkyls, secondary alkyls, tertiary alkyls, hetero atoms, and alkyl groups bearing hetero atoms;
where at least one of X3 or X4 is selected from the group consisting of aryls, primary alkyls, secondary alkyls, tertiary alkyls, hetero atoms, and alkyl groups bearing hetero atoms; and
where Y1, Y2, Y3, Y4, Y5, Y6, Z1, Z2, Z3, Z4, Z5, Z6, Z7, Z8, Z9, Z10, Z11, and Z12 are independently selected from the group consisting of hydrogen, halides, alkyls, aryls, alkyl groups bearing hetero atoms, and hetero atoms.

21. The catalyst of Claim 20 wherein the transition metal ion is selected from the group consisting of Mn, Cr, Fe, Ni, Co, Ti, V, Ru, and Os.

22. The catalyst of Claim 20 wherein the transition metal ion is selected from the group consisting of Mn, Cr, Fe, Ni, and Co.

23. The catalyst of Claim 20 wherein Y1, Y2, Y3, Y4, Y5, Y6, Z1, Z2, Z3, Z4, Z5, Z6, Z7, Z8, Z9, Z10, Z11, and Z12 are hydrogen.

24. The catalyst of Claim 20 wherein X1 and X3 are independently selected from the group consisting of t-butyl and phenyl.

25. The catalyst of Claim 20 wherein X1 and X3 are the same.

26. A chiral catalyst having the general formula (III): 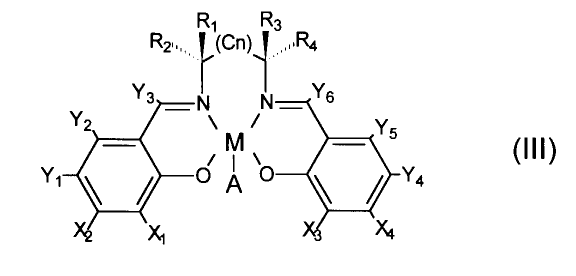 where M is a transition metal ion;
where A is an anion;
where n is either 0, 1, or 2;
where at least one of X1 or X2 is selected from the group consisting of aryls, primary alkyls, secondary alkyls, tertiary alkyls, hetero atoms, and alkyl groups bearing hetero atoms;
where at least one of X3 or X4 is selected from the group consisting of aryls, primary alkyls, secondary alkyls, tertiary alkyls, hetero atoms, and alkyl groups bearing hetero atoms;
where at least one of Y1 or Y2 is selected from the group consisting of aryls, primary alkyls, secondary alkyls, tertiary alkyls, hetero atoms, and alkyl groups bearing hetero atoms;
where at least one of Y4 or Y5 is selected from the group consisting of aryls, primary alkyls, secondary alkyls, tertiary alkyls, hetero atoms, and alkyl groups bearing hetero atoms;
where Y3 and Y6 are independently selected from the group consisting of H and primary alkyl groups;
where either one or two of R1, R2, R3 and R4 is hydrogen;
where, if R1 is hydrogen, then R3 is a primary alkyl;
where, if R2 is hydrogen, then R4 is a primary alkyl;
where, if R3 is hydrogen, then R1 is a primary alkyl;
where, if R4 is hydrogen, then R2 is a primary alkyl; and where the carbons in the (Cₙ) portion have substituents selected from the group consisting of hydrogen, alkyl, aryl, and hetero atoms.

27. The catalyst of Claim 26 wherein the transition metal ion is selected from the group consisting of Mn, Cr, Fe, Ni, Co, Ti, V, Ru, and Os.

28. The catalyst of Claim 26 wherein the metal ion is Mn.

29. The catalyst of Claim 26 wherein R1 is the same as R3 and R2 is the same as R4.

30. The catalyst of Claim 26 wherein X1 and X3 are independently selected from the group consisting of t-butyl and phenyl.

31. The catalyst of Claim 30 wherein X1 and X3 are the same.

32. The catalyst of Claim 30 wherein Y1 and Y4 are independently selected from the group consisting of t-butyl and phenyl.

33. The catalyst of Claim 32 wherein Y1 and Y4 are the same.

34. The catalyst of Claim 26 wherein X1, X3, Y1 and Y4 are independently selected from the group consisting of t-butyl and phenyl.

35. The catalyst of Claim 34 wherein X1, X3, Y1 and Y4 are all the same.

36. The catalyst of Claim 26 wherein X1, X3, Y1 and Y4 are all t-butyl.

37. The catalyst of Claim 26 wherein R1 and R4 are hydrogen and R2 and R3 are methyl.

38. A chiral catalyst having the general formula (IV) : 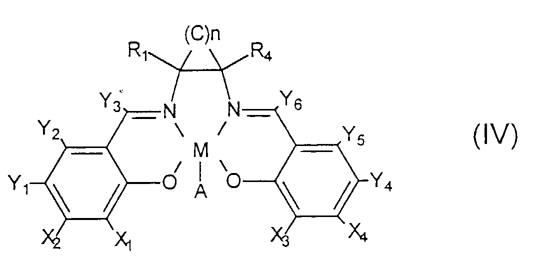
where M is a transition metal ion;
where A is an anion;
where n is either 3, 4, 5 or 6;
where at least one of X1 or X2 is selected from the group consisting of aryls, primary alkyls, secondary alkyls, tertiary alkyls, hetero atoms, and alkyl groups bearing hetero atoms;
where at least one of X3 or X4 is selected from the group consisting of aryls, primary alkyls, secondary alkyls, tertiary alkyls, hetero atoms, and alkyl groups bearing hetero atoms;
where at least one of Y1 or Y2 is selected from the group consisting of aryls, primary alkyls, secondary alkyls, tertiary alkyls, hetero atoms, and alkyl groups bearing hetero atoms;
where at least one of Y4 or Y5 is selected from the group consisting of aryls, primary alkyls, secondary alkyls, tertiary alkyls, hetero atoms, and alkyl groups bearing hetero atoms;
where Y3 and Y6 are independently selected from the group consisting of hydrogen and primary alkyl groups;
where R1 and R4 are trans to each other and are selected from the group consisting of primary alkyls and hydrogen; and
where the carbons in the (C)ₙ portion have substituents selected from the group consisting of hydrogen, alkyl, aryl, and heteroatoms.

39. The catalyst of Claim 38 wherein the transition metal ion is selected from the group consisting of Mn, Cr, Fe, Ni, Co, Ti, V, Ru, and Os.

40. The catalyst of Claim 38 wherein the metal ion is Mn.

41. The catalyst of Claim 38 wherein R1 is the same as R4.

42. The catalyst of Claim 38 wherein X1 and X3 are independently selected from the group consisting of t-butyl and phenyl.

43. The catalyst of Claim 42 wherein X1 and X3 are the same.

44. The catalyst of Claim 38 wherein Y1 and Y4 are independently selected from the group consisting of t-butyl and phenyl.

45. The catalyst of Claim 44 wherein Y1 and Y4 are the same.

46. The catalyst of Claim 38 wherein X1, X3, Y1 and Y4 are independently selected from the group consisting of t-butyl and phenyl.

47. The catalyst of Claim 46 wherein X1, X3, Y1 and Y4 are all the same.

48. The catalyst of Claim 38 wherein X1, X3, Y1 and Y4 are all t-butyl.

49. The catalyst of Claim 48 wherein R1 and R4 are hydrogen.

50. The catalyst of Claim 38 wherein R1 and R4 are selected from the group consisting of hydrogen and methyl.

51. The catalyst of Claim 38 wherein R1 and R4 are hydrogen.

52. The catalyst of Claim 38 wherein n is 4.

53. A chiral catalyst according to claim 38 having the general formula (IVa) : 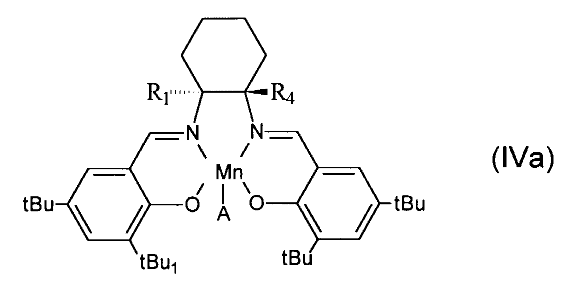
where A is an anion; and
where R1 and R4 are trans to each other and are selected from the group consisting of primary alkyls and hydrogen.

54. The catalyst of Claim 53 wherein R1 is the same as R4.

55. The catalyst of Claim 54 wherein R1 and R4 are hydrogen.

56. The catalyst of Claim 53 wherein R1 and R4 are selected from the group consisting of hydrogen and methyl.

57. A method of enantioselectively epoxidizing a prochiral olefin with the use of a chiral catalyst comprising the steps of:
providing a prochiral olefin;
providing an oxygen atom source;
providing the chiral catalyst of Claim 1, 20, 26, 38 or 53, and
reacting said olefin, said oxygen atom source, and said chiral catalyst under such conditions and for such time sufficient to epoxidize said olefin.

58. The method of Claim 57 wherein the prochiral olefin is selected from the group consisting of monosubstituted, cis-1,2-disubstituted and cyclic olefins.

59. The method of Claim 57 wherein the prochiral olefin is a cis disubstituted olefin bearing a primary substitutent on one side of the double bond and a secondary, tertiary, or aryl substituent on the other side.

60. The method of Claim 57 wherein the prochiral olefin is selected from the group consisting of:
cis-β-methylstyrene, dihydronaphthalene, 2cyclohexenyl-1,1-dioxolane, 2,2-dimethylchromene, styrene, and propylene.

61. The method of Claim 57 wherein the oxygen atom source is selected from the group consisting of: NaOCl, iodosylmesitylene, NaIO4, NBu₄IO₄, potassium peroxymonosulfate, magnesium monoperoxyphthalate, and hexacyanoferrate ions.

62. The method of Claim 57 wherein the oxygen atom source is selected from group consisting of: NaOCl and iodosylmesitylene.

63. The method of Claim 57 wherein the oxygen atom source is NaOCl.

## Patentansprüche

1. Ein chiraler Katalysator der allgemeinen Formel (I) 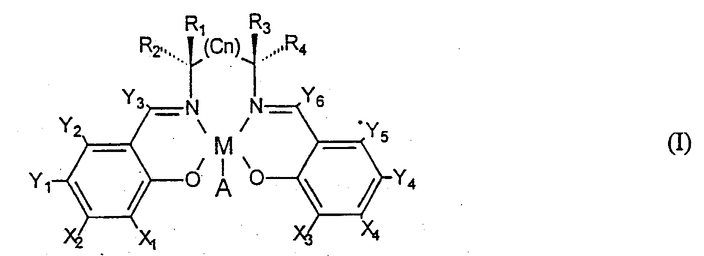 in der M ein Übergangsmetallion ist, A ein Anion ist, n den Wert 0, 1 oder 2 hat, mindestens einer der Reste X1 oder X2 ausgewählt ist aus der Gruppe bestehend aus Arylresten, primären Alkylresten, sekundären Alkylresten, tertiären Alkylresten, Heteroatomen und Heteroatome tragenden Alkylresten, mindestens einer der Reste X3 oder X4 ausgewählt ist aus der Gruppe bestehend aus Arylresten, primären Alkylresten, sekundären Alkylresten, tertiären Alkylresten, Heteroatomen und Heteratome tragenden Alkylresten, Y3 und Y6 unabhängig ausgewählt sind aus der Gruppe bestehend aus Wasserstoffatomen, Halogenatomen, Alkylresten, Arylresten und Heteroatome tragenden Alkylresten, und Y1, Y2, Y4 und Y5 unabhängig ausgewählt sind aus der Gruppe bestehend aus Wasserstoffatomen, Halogenatomen, Alkylresten, Arylresten, Heterome tragenden Alkylresten, Alkoxyresten und Nitrogruppen, mindestens einer der Reste R1, R2, R3 und R4 ausgewählt ist aus einer ersten Gruppe bestehend aus Wasserstoffatomen, Methylgruppen, Ethylgruppen und primären Alkylresten, wobei, sofern R1 ausgewählt ist aus der ersten Gruppe, R2 und R3 ausgewählt sind aus einer zweiten Gruppe bestehend aus Arylresten, sekundären Alkylresten, tertiären Alkylresten und Heteroatome tragenden Alkylresten, wobei, wenn R2 ausgewählt ist aus der ersten Gruppe, R1 und R4 ausgewählt sind aus der zweiten Gruppe, wobei, wenn R3 ausgewählt ist aus der ersten Gruppe, R1 und R4 ausgewählt sind aus der zweiten Gruppe, wobei, wenn R4 ausgewählt ist aus der ersten Gruppe, R2 und R3 ausgewählt sind aus der zweiten Gruppe, und wobei die Kohlenstoffatome im (Cₙ)-Teil Substituenten tragen ausgewählt aus der Gruppe bestehend aus Wasserstoffatomen, Alkylresten, Arylresten und Heteroatomen.

2. Der Katalysator nach Anspruch 1, wobei das Übergangsmetallion ausgewählt ist aus der Gruppe bestehend aus Mn, Cr, Fe, Ni, Co, Ti, V, Ru und Os.

3. Der Katalysator nach Anspruch 1, wobei das Übergangsmetallion ausgewählt ist aus der Gruppe bestehend aus Mn, Cr, Fe, Ni und Co.

4. Der Katalysator nach Anspruch 1, wobei das Metallion Mn ist.

5. Der Katalysator nach Anspruch 1, wobei die erste Gruppe aus Wasserstoffatomen und Methylgruppen besteht.

6. Der Katalysator nach Anspruch 1, wobei die erste Gruppe aus Wasserstoffatomen besteht.

7. Der Katalysator nach Anspruch 1, wobei die zweite Gruppe aus tert.-Butyl- und Phenylgruppen besteht.

8. Der Katalysator nach Anspruch 1, wobei die zweite Gruppe aus Phenylgruppen besteht.

9. Der Katalysator nach Anspruch 1, wobei R1 der gleiche Rest ist wie R4 und R2 der gleiche Rest ist wie R3.

10. Der Katalysator nach Anspruch 9, wobei die erste Gruppe aus Wasserstoffatomen und Methylgruppen besteht.

11. Der Katalysator nach Anspruch 9, wobei die zweite Gruppe aus tert.-Butyl- und Phenylgruppen besteht.

12. Der Katalysator nach Anspruch 1, wobei X1 und X3 unabhängig ausgewählt sind aus der Gruppe bestehend aus tert.-Butyl- und Phenylgruppen.

13. Der Katalysator nach Anspruch 1, wobei X1 und X3 gleich sind.

14. Der Katalysator nach Anspruch 1, wobei X1 und X3 tert.-Butylgruppen sind.

15. Der Katalysator nach Anspruch 9, wobei X1 und X3 gleich sind und ausgewählt sind aus der Gruppe bestehend aus Arylresten, primären Alkylresten, sekundären Alkylresten, tertiären Alkylresten, Heteroatomen und Heteroatome tragenden Alkylresten.

16. Der Katalysator nach Anspruch 15, wobei das Metallion Mangan ist.

17. Der Katalysator nach Anspruch 15, wobei die erste Gruppe aus Wasserstoffatomen besteht.

18. Der Katalysator nach Anspruch 15, wobei die zweite Gruppe aus tert.-Butylgruppen und Phenylgruppen besteht.

19. Der Katalysator nach Anspruch 15, wobei die zweite Gruppe aus Phenylgruppen besteht.

20. Ein chiraler Katalysator der allgemeinen Formel (II) 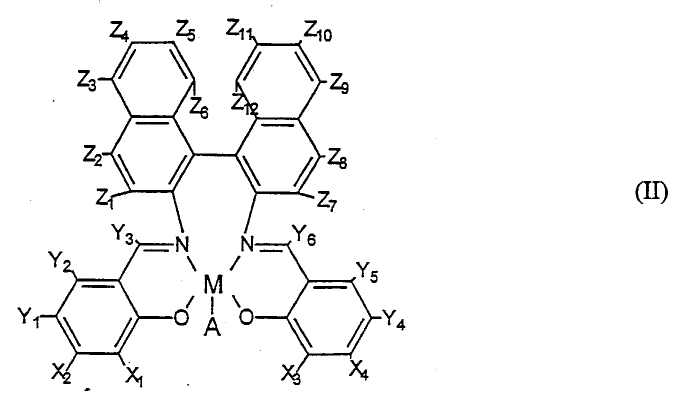 in der M ein Übergangsmetallion ist, A ein Anion ist, mindestens einer der Reste X1 oder X2 ausgewählt ist aus der Gruppe bestehend aus Arylresten, primären Alkylresten, sekundären Alkylresten, tertiären Alkylresten, Heteroatomen und Heteroatome tragenden Alkylresten, mindestens einer der Reste X3 oder X4 ausgewählt ist aus der Gruppe bestehend aus Arylresten, primären Alkylresten, sekundären Alkylresten, tertiären Alkylresten, Heteroatomen und Heteroatome tragenden Alkylresten, und Y1, Y2, Y3, Y4, Y5, Y6, Z1, Z2, Z3, Z4, Z5, Z6, Z7, Z8, Z9, Z10, Z11 und Z12 unabhängig ausgewählt sind aus der Gruppe bestehend aus Wasserstoffatomen, Halogenatomen, Alkylresten, Arylresten, Heteroatome tragenden Alkylresten und Heteroatomen.

21. Der Katalysator nach Anspruch 20, wobei das Übergangsmetallion ausgewählt ist aus der Gruppe bestehend aus Mn, Cr, Fe, Ni, Co, Ti, V, Ru und Os.

22. Der Katalysator nach Anspruch 20, wobei das Übergangsmetallion ausgewählt ist aus der Gruppe bestehend aus Mn, Cr, Fe, Ni und Co.

23. Der Katalysator nach Anspruch 20, wobei Y1, Y2, Y3, Y4, Y5, Y6, Z1, Z2, Z3, Z4, Z5, Z6, Z7, Z8, Z9, Z10, Z11 und Z12 Wasserstoffatome sind.

24. Der Katalysator nach Anspruch 20, wobei X1 und X3 unabhängig ausgewählt sind aus der Gruppe bestehend aus tert.-Butylgruppen und Phenylgruppen.

25. Der Katalysator nach Anspruch 20, wobie X1 und X3 gleich sind.

26. Ein chiraler Katalysator der allgemeinen Formel (III) 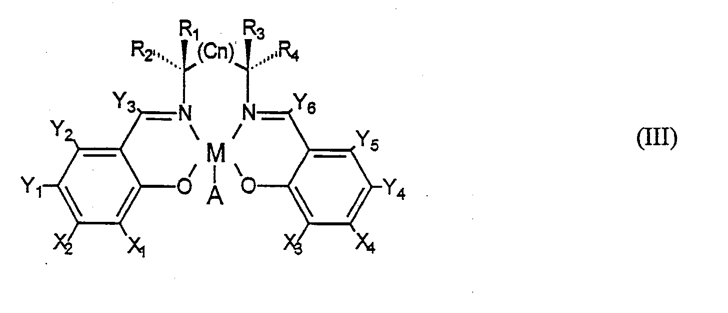 in der M ein Übergangsmetallion ist, A ein Anion ist, n den Wert 0, 1 oder 2 hat, mindestens einer der Reste X1 oder X2 ausgewählt ist aus der Gruppe bestehend Arylresten, primären Alkyresten, sekundären Alkylresten, tertiären Alkylresten, Heteroatomen und Heteroatome tragenden Alkyresten, mindestens einer der Reste X3 oder X4 ausgewählt ist aus der Gruppe bestehend aus Arylresten, primären Alkylresten, sekundären Alkylresten, tertiären Alkylresten, Heteroatomen und Heteroatome tragende Alkylresten, mindestens einer der Reste Y1 oder Y2 ausgewählt ist aus der Gruppe bestehend aus Arylresten, primären Alkylresten, sekundären Alkylresten, tertiären Alkylresten, Heteroatomen und Heteroatome tragenden Alkylresten, mindestens einer der Reste Y4 oder Y5 ausgewählt ist aus der Gruppe bestehend aus Arylresten, primären Alkylresten, sekundären Alkylresten, tertiären Alkylresten, Heteroatomen und Heteroatome tragenden Alkylresten, Y3 und Y6 unabhängig ausgewählt sind aus der Gruppe bestehend aus Wasserstoffatomen und primären Alkylresten, entweder einer oder zwei Reste R1, R2, R3 und R4 Wasserstoffatome sind, wobei, wenn R1 ein Wasserstoffatom ist, R3 ein primärer Alkylrest ist, wobei, wenn R2 ein Wasserstoffatom ist, R4 ein primärer Alkylrest ist, wobei, wenn R3 ein Wasserstoffatom, R1 ein primärer Alkylrest ist, wobei, wenn R4 ein Wasserstoffatom ist, R2 ein primärer Alkylrest ist und wobei die Kohlenstoffatome im (Cₙ)-Teil Substituenten tragen ausgewählt aus der Gruppe bestehend aus Wasserstoffatomen, Alkylresten, Arylresten und Heteroatomen.

27. Der Katalysator nach Anspruch 26, wobei das Übergangsmetallion ausgewählt ist aus der Gruppe bestehend aus Mn, Cr, Fe, Ni, Co, Ti, V, Ru und Os.

28. Der Katalysator nach Anspruch 26, wobei das Metallion Mn ist.

29. Der Katalysator nach Anspruch 26, wobei R1 der gleiche Rest ist wie R3 und R2 der gleiche Rest ist wie R4.

30. Der Katalysator nach Anspruch 26, wobei X1 und X3 unabhängig ausgewählt sind aus der Gruppe bestehend aus tert.-Butyl- und Phenylgruppen.

31. Der Katalysator nach Anspruch 30, wobei X1 und X3 gleich sind.

32. Der Katalysator nach Anspruch 30, wobei Y1 und Y4 unabhängig ausgewählt sind aus der Gruppe bestehend aus tert.-Butyl- und Phenylgruppen.

33. Der Katalysator nach Anspruch 32, wobei Y1 und Y4 gleich sind.

34. Der Katalysator nach Anspruch 26, wobei X1, X3, Y1 und Y4 unabhängig ausgewählt sind aus der Gruppe bestehend aus tert.-Butyl- und Phenylgruppen.

35. Der Katalysator nach Anspruch 34, wobei X1, X3, Y1 und Y4 alle gleich sind.

36. Der Katalysator nach Anspruch 26, wobei X1, X3, Y1 und Y4 alle tert.-Butylgruppen sind.

37. Der Katalysator nach Anspruch 26, wobei R1 und R4 Wasserstoffatome und R2 und R3 Methylgruppen sind.

38. Ein chiraler Katalysator der allgemeinen Formel (IV) 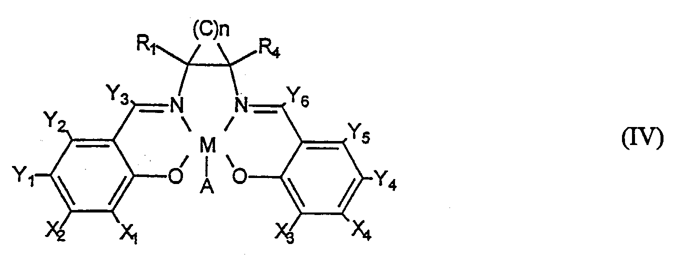 in der M ein Übergangsmetallion ist, A ein Anion ist, n den Wert 3, 4, 5 oder 6 hat, mindestens einer der Reste X1 oder X2 ausgewählt ist aus der Gruppe bestehend aus Arylresten, primären Alkylresten, sekundären Alkylresten, tertiären Alkylresten, Heteroatomen und Heteroatome tragenden Alkylresten, mindestens einer der Reste X3 oder X4 ausgewählt ist aus der Gruppe bestehend aus Arylresten, primären Alkylresten, sekundären Alkylresten, tertiären Alkylresten, Heteroatomen und Heteroatome tragenden Alkylresten, mindestens einer der Rest Y1 oder Y2 ausgewählt ist aus der Gruppe bestehend aus Arylresten, primären Alkylresten, sekundären Alkylresten, tertiären Alkylresten, Heteroatomen und Heteroatome tragenden Alkylresten, mindestens einer der Reste Y4 oder Y5 ausgewählt ist aus der Gruppe bestehend aus Arylresten, primären Alkylresten, sekundären Alkylresten, tertiären Alkylresten, Heteroatomen und Heteroatome tragenden Alkylresten, Y3 und Y6 unabhängig ausgewählt sind aus der Gruppe bestehend aus Wasserstoffatomen und primären Alkylresten, R1 und R4 zueinander in Transstellung stehen und ausgewählt sind aus der Gruppe bestehend aus primären Alkylresten und Wasserstoffatomen und die Kohlenstoffatome im (C)ₙ-Teil Substituenten tragen ausgewählt aus der Gruppe bestehend aus Wasserstoffatomen, Alkylresten, Arylresten und Heteroatomen.

39. Der Katalysator nach Anspruch 38, wobei das Übergangsmetallion ausgewählt ist aus der Gruppe bestehend aus Mn, Cr, Fe, Ni, Co, Ti, V, Ru und Os.

40. Der Katalysator nach Anspruch 38, wobei das Metallion Mn ist.

41. Der Katalysator nach Anspruch 38, wobei R1 der gleiche Rest ist wie R4.

42. Der Katalysator nach Anspruch 38, wobei X1 und X3 unabhängig ausgewählt sind aus der Gruppe bestehend aus tert.-Butyl- und Phenylgruppen.

43. Der Katalysator nach Anspruch 42, wobei X1 und X3 gleich sind.

44. Der Katalysator nach Anspruch 38, wobei Y1 und Y4 unabhängig ausgewählt sind aus der Gruppe bestehend aus tert.-Butyl- und Phenylgruppen.

45. Der Katalysator nach Anspruch 44, wobei Y1 und Y4 gleich sind.

46. Der Katalysator nach Anspruch 38, wobei X1, X3, Y1 und Y4 unabhängig ausgewählt sind aus der Gruppe bestehend aus tert.-Butyl- und Phenylgruppen.

47. Der Katalysator nach Anspruch 46, wobei X1, X3, Y1 und Y4 alle gleich sind.

48. Der Katalysator nach Anspruch 38, wobei X1, X3, Y1 und Y4 alle tert.-Butylgruppen sind.

49. Der Katalysator nach Anspruch 48, wobei Rl und R4 Wasserstoffatome sind.

50. Der Katalysator nach Anspruch 38, wobei R1 und R4 ausgewählt sind aus der Gruppe bestehend aus Wasserstoffatomen und Methylgruppen.

51. Der Katalysator nach Anspruch 38, wobei R1 und R4 Wasserstoffatome sind.

52. Der Katalysator nach Anspruch 38, wobei n den Wert 4 hat.

53. Ein chiraler Katalysator nach Anspruch 38 der allgemeinen Formel (IVa) 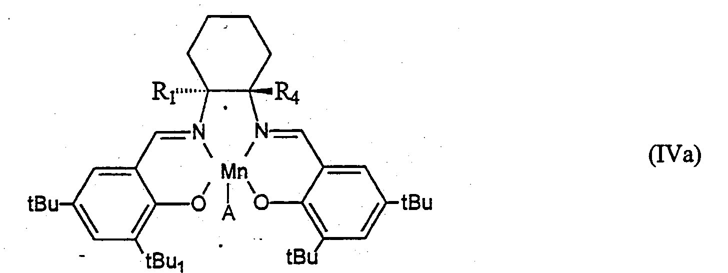 in der A ein Anion ist und R1 und R4 in Transstellung zueinander stehen und ausgewählt sind aus der Gruppe bestehend aus primären Alkylresten und Wasserstoffatomen.

54. Der Katalysator nach Anspruch 53, wobei R1 der gleiche Rest ist wie R4.

55. Der Katalysator nach Anspruch 54, wobei R1 und R4 Wasserstoffatome sind.

56. Der Katalysator nach Anspruch 53, wobei Rl und R4 ausgewählt sind aus der Gruppe bestehend aus Wasserstoffatomen und Methylgruppen.

57. Verfahren zur enantioselektiven Epoxidation eines prochiralen Olefins unter Verwendung eines chiralen Katalysators umfassend die Schritte:
Bereitstellung eines prochiralen Olefins;
Bereitstellung einer Sauerstoffatom-Quelle;
Bereitstellung des chiralen Katalysators nach Anspruch 1, 20, 26, 38 oder 53 und
Umsetzung des Olefins mit der Sauerstoffatom-Quelle und dem chiralen Katalysator unter solchen Bedingungen und für eine ausreichende Zeit zur Epoxidation des Olefins.

58. Verfahren nach Anspruch 57, wobei das prochirale Olefin ausgewählt ist aus der Gruppe bestehend aus monosubstituierten, cis-1,2-disubstituierten und cyclischen Olefinen.

59. Verfahren nach Anspruch 57, wobei das prochirale Olefin ein cis-disubstituiertes Olefin mit einem primären Substituenten an einer Seite der Doppelbindung und einem sekundären, tertiären oder Arylsubstituenten an der anderen Seite ist.

60. Verfahren nach Anspruch 57, wobei das prochirale Olefin ausgewählt ist aus der Gruppe bestehend aus cis-ß-Methylstyrol, Dihydronaphthalin, 2-Cyclohexenyl-1,1-dioxolan, 2,2-Dimethylchromen, Styrol und Propylen.

61. Verfahren nach Anspruch 57, wobei die Sauerstoffatom-Quelle ausgewählt ist aus der Gruppe bestehend aus NaOCl, Iodosylmesitylen, NaJO4, NBu₄JO₄, Kaliumperoximonosulfat, Magnesiummonoperoxyphthalat und Hexacyanoferrat-Ionen.

62. Verfahren nach Anspruch 57, wobei die Sauerstoffatom-Quelle ausgewählt ist aus der Gruppe bestehend aus NaOCl und Iodosylmesitylen.

63. Verfahren nach Anspruch 57, wobei die Sauerstoffatom-Quelle NaOCl ist.

## Revendications

1. Catalyseur chiral ayant la formule générale (I): 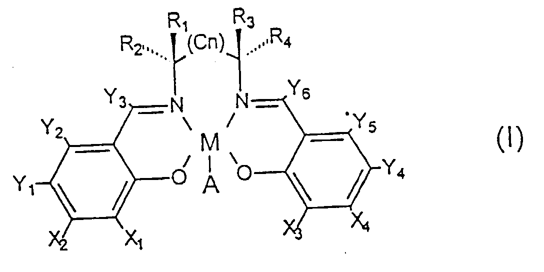
où M est un ion de métal de tansition;
où A est un anion;
où n est 0, 1 ou 2;
où au moins un des X1 ou X2 est choisi dans le groupe consistant en des aryles, des alkyles primaires, des alkyles secondaires, des alkyles tertiaires, des hétéroatomes et des groupes alkyle portant des hétéroatomes;
où au moins un des X3 ou X4 est choisi dans le groupe consistant en des aryles, des alkyles primaires, des alkyles secondaires, des alkyles tertiaires, des hétéroatomes et des groupes alkyle portant des hétéroatomes;
où Y3 et Y6 sont, indépendamment l'un de l'autre, choisis dans le groupe consistant en l'hydrogène, des halogénures, des alkyles, des aryles et des groupes alykle portant des hétéroatomes, et Y1, Y2, Y4 et Y5 sont choisis indépendamment dans le groupe consistant en l'hydrogène, des halogénures, des alkyles, des aryles, des groupes alkyle portant des hétéroatomes, des groupes alcoxy et des groupes nitro;
où au moins un des R1, R2, R3 et R4 est choisi dans un premier groupe consistant en l'hydrogène, CH₃, C₂H₅ et des alkyles primaires;
où, si R1 est choisi dans ledit premier groupe, alors R2 et R3 sont choisis dans un second groupe consistant en des aryles, des alkyles secondaires, des alkyles tertiaires et des groupes alkyle portant des hétéroatomes;
où, si R2 est choisi dans ledit premier groupe, alors R1 et R4 sont choisis dans ledit second groupe;
où, si R3 est choisi dans ledit premier groupe, alors R1 et R4 sont choisis dans ledit second groupe;
où, si R4 est choisi dans ledit premier groupe, alors R2 et R3 sont choisis dans ledit second groupe; et où les atomes de carbone dans la portion (Cn) ont des substituants choisis dans le groupe consistant en l'hydrogène, un alkyle, un aryle et des hétéroatomes.

2. Catalyseur selon la revendication 1, dans lequel l'ion de métal de transition est choisi dans le groupe consistant en Mn, Cr, Fe, Ni, Co, Ti, V, Ru et Os.

3. Catalyseur selon la revendication 1, dans lequel l'ion de métal de transition est choisi dans le groupe consistant en Mn, Cr, Fe, Ni et Co.

4. Catalyseur selon la revendication 1, dans lequel l'ion de métal est Mn.

5. Catalyseur selon la revendication 1, dans lequel ledit premier groupe consiste en de l'hydrogène et du méthyle.

6. Catalyseur selon la revendication 1, dans lequel ledit premier groupe consiste en de l'hydrogène.

7. Catalyseur selon la revendication 1, dans lequel ledit second groupe consiste en du t-butyle et du phényle.

8. Catalyseur selon la revendication 1, dans lequel ledit second groupe consiste en du phényle.

9. Catalyseur selon la revendication 1, dans lequel R1 est identique à R4 et R2 est identique à R3.

10. Catalyseur selon la revendication 9, dans lequel ledit premier groupe consiste en de l'hydrogène et du méthyle.

11. Catalyseur selon la revendication 9, dans lequel ledit second groupe consiste en du t-butyle et du phényle.

12. Catalyseur selon la revendication 1, dans lequel X1 et X3 sont choisis indépendamment dans le groupe consistant en du t-butyle et du phényle.

13. Catalyseur selon la revendication 12, dans lequel X1 et X3 sont identiques.

14. Catalyseur selon la revendication 1, dans lequel à la fois X1 et X3 sont du t-butyle.

15. Catalyseur selon la revendication 9, dans lequel:
X1 et X3 sont identiques et sont choisis dans le groupe consistant en des aryles, des alkyles primaires, des alkyles secondaires, des alkyles tertiaires, des hétéroatomes et des groupes alkyle portant des hétéroatomes.

16. Catalyseur selon la revendication 15, dans lequel l'ion métal est le manganèse.

17. Catalyseur selon la revendication 15, dans lequel ledit premier groupe consiste en de l'hydrogène.

18. Catalyseur selon la revendication 15, dans lequel ledit second groupe consiste en du t-butyle et du phényle.

19. Catalyseur selon la revendication 15, dans lequel ledit second groupe consiste en du phényle.

20. Catalyseur chiral ayant la formule générale (II) : 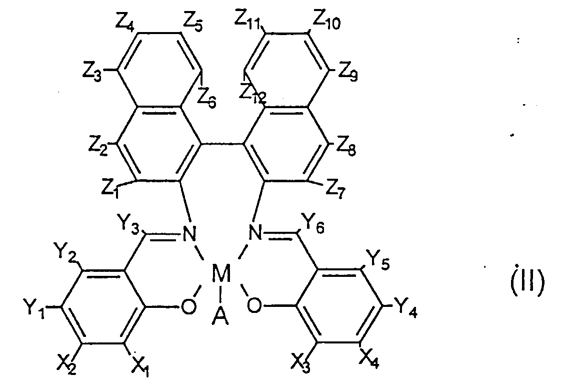
où M est un ion de métal de transition;
où A est un anion;
où au moins l'un des X1 ou X2 est choisi dans le groupe consistant en des aryles, des alkyles primaires, des alkyles secondaires, des alkyles tertiaires, des hétéroatomes et des groupes alkyle portant des hétéroatomes ;
où au moins l'un des X3 ou X4 est choisi dans le groupe consistant en des aryles, des alkyles primaires, des alkyles secondaires, des alkyles tertiaires, des hétéroatomes et des groupes alkyle portant des hétéroatomes; et
où Y1, Y2, Y3, Y4, Y5, Y6, Z1, Z2, Z3, Z4, Z5, Z6, Z7, Z8, Z9, Z10, Z11 et Z12 sont choisis indépendamment dans le groupe consistant en l'hydrogène, des halogénures, des alkyles, des aryles, des groupes alkyle portant des hétéroatomes et des hétéroatomes.

21. Catalyseur selon la revendication 20, dans lequel l'ion de métal de transition est choisi dans le groupe consistant en Mn, Cr, Fe, Ni, Co, Ti, V, Ru et Os.

22. Catalyseur selon la revendicaton 20, dans lequel l'ion de métal de transition est choisi dans le groupe consistant en Mn, Cr, Fe, Ni et Co.

23. Catalyseur selon la revendication 20, dans lequel Y1, Y2, Y3, Y4, Y5, Y6, Z1, Z2, Z3, Z4, Z5, Z6, Z7, Z8, Z9, Z10, Z11 et Z12 sont l'hydrogène.

24. Catalyseur selon la revendication 20, dans lequel X1 et X3 sont choisis indépendamment dans le groupe consistant en du t-butyle et du phényle.

25. Catalyseur selon la revendication 20, dans lequel X1 et X3 sont identiques.

26. Catalyseur chiral ayant la formule générale (III): 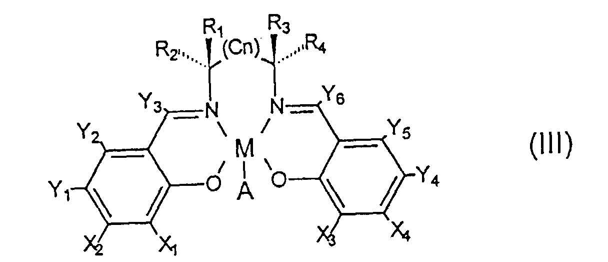
où M est un ion de métal de transition;
où A est un anion;
où n est 0, 1 ou 2;
où au moins l'un des X1 ou X2 est choisi dans le groupe consistant en des aryles, des alkyles primaires, des alkyles secondaires, des alkyles tertiaires, des hétéroatomes et des groupes alkyle portant des hétéroatomes;
où au moins l'un des X3 ou X4 est choisi dans le groupe consistant en des aryles, des alkyles primaires, des alkyles secondaires, des alkyles tertiaires, des hétéroatomes et des groupes alkyle portant des hétéroatomes;
où au moins l'un des Y1 ou Y2 est choisi dans le groupe consistant en des aryles, des alkyles primaires, des alkyles secondaires, des alkyles tertiaires, des hétéroatomes et des groupes alkyle portant des hétéroatomes;
où au moins l'un des Y4 ou Y5 est choisi dans le groupe consistant en des aryles, des alkyles primaires, des alkyles secondaires, des alkyles tertiaires, des hétéroatomes et des groupes alkyle portant des hétéroatomes;
où Y3 et Y6 sont choisis indépendamment dans le groupe consistant en H et des groupes alkyle primaire;
où soit l'un soit deux des R1, R2, R3 et R4 est l'hydrogène;
où, si R1 est l'hydrogène, alors R3 est un alkyle primaire;
où, si R2 est l'hydrogène, alors R4 est un alkyle primaire;
où, si R3 est l'hydrogène, alors R1 est un alkyle primaire;
où, si R4 est l'hydrogène, alors R2 est un alkyle primaire; et où les carbones dans la portion (Cₙ) ont des substituants choisis dans le groupe consistant en l'hydrogène, un alkyle, un aryle et des hétéroatomes.

27. Catalyseur selon la revendication 26, dans lequel l'ion de métal de transition est choisi dans le groupe consistant en Mn, Cr, Fe, Ni, Co, Ti, V, Ru et Os.

28. Catalyseur selon la revendication 26, dans lequel l'ion de métal est Mn.

29. Catalyseur selon la revendication 26, dans lequel R1 est identique à R3 et R2 est identique à R4.

30. Catalyseur selon la revendication 26, dans lequel X1 et X3 sont choisis indépendamment dans le groupe consistant en du t-butyle et du phényle.

31. Catalyseur selon la revendication 30, dans lequel X1 et X3 sont identiques.

32. Catalyseur selon la revendication 30, dans lequel Y1 et Y4 sont choisis indépendamment dans le groupe consistant en du t-butyle et du phényle.

33. Catalyseur selon la revendication 32, dans lequel Y1 et Y4 sont identiques.

34. Catalyseur selon la revendication 26, dans lequel X1, X3, Y1 et Y4 sont choisis indépendamment dans le groupe consistant en du t-butyle et du phényle.

35. Catalyseur selon la revendication 34, dans lequel X1, X3, Y1 et Y4 sont tous identiques.

36. Catalyseur selon la revendication 26, dans lequel X1, X3, Y1 et Y4 sont tous du t-butyle.

37. Catalyseur selon la revendication 26, dans lequel R1 et R4 sont l'hydrogène et R2 et R3 sont du méthyle.

38. Catalyseur chiral ayant la formule générale (IV): 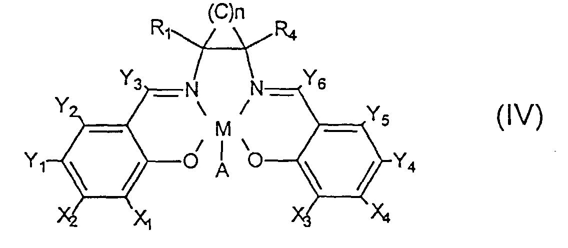
où M est un ion de métal de transition;
où A est un anion;
où n est 3, 4, 5 ou 6;
où au moins l'un de X1 ou X2 est choisi dans le groupe consistant en des aryles, des alkyles primaires, des alkyles secondaires, des alkyles tertiaires, des hétéroatomes et des groupes alkyle portant des hétéroatomes;
où au moins l'un des X3 ou X4 est choisi dans le groupe consistant en des aryles, des alkyles primaires, des alkyles secondaires, des alkyles tertiaires, des hétéroatomes et des groupes alkyle portant des hétéroatomes;
où au moins l'un des Y1 ou Y2 est choisi dans le groupe consistant en des aryles, des alkyles primaires, des alkyles secondaires, des alkyles tertiaires, des hétéroatomes et des groupes alkyle portant des hétéroatomes;
où au moins l'un des Y4 ou Y5 est choisi dans le groupe consistant en des aryles, des alkyles primaires, des alkyles secondaires, des alkyles tertiaires, des hétéroatomes et des groupes alkyle portant des hétéroatomes;
où Y3 et Y6 sont choisis indépendamment dans le groupe consistant en l'hydrogène et des groupes alkyle primaire;
où R1 et R4 sont trans l'un par rapport à l'autre et sont choisis dans le groupe consistant en des alkyles primaires et de l'hydrogène; et
où les carbones dans la portion (Cₙ) ont des substituants choisis dans le groupe consistant en l'hydrogène, un alkyle, un aryle et des hétéroatomes.

39. Catalyseur selon la revendication 38, dans lequel l'ion de métal de transition est choisi dans le groupe consistant en Mn, Cr, Fe, Ni, Co, Ti, V, Ru et Os.

40. Catalyseur selon la revendication 38, dans lequel l'ion de métal est Mn.

41. Catalyseur selon la revendication 38, dans lequel R1 est identique à R4.

42. Catalyseur selon la revendication 38, dans lequel X1 et X3 sont choisis indépendamment dans le groupe consistant en du t-butyle et du phényle.

43. Catalyseur selon la revendication 42, dans lequel X1 et X3 sont identiques.

44. Catalyseur selon la revendication 38, dans lequel Y1 et Y4 sont choisis indépendamment dans le groupe consistant en du t-butyle et du phényle.

45. Catalyseur selon la revendication 44, dans lequel Y1 et Y4 sont identiques.

46. Catalyseur selon la revendication 38, dans lequel X1, X3, Y1 et Y4 sont choisis indépendamment dans le groupe consistant en du t-butyle et du phényle.

47. Catalyseur selon la revendication 46, dans lequel X1, X3, Y1 et Y4 sont tous identiques.

48. Catalyseur selon la revendication 38, dans lequel X1, X3, Y1 et Y4 sont tous du t-butyle.

49. Catalyseur selon la revendication 48, dans lequel R1 et R4 sont l'hydrogène.

50. Catalyseur selon la revendication 38, dans lequel R1 et R4 sont choisis dans le groupe consistant en de l'hydrogène et du méthyle.

51. Catalyseur selon la revendication 38, dans lequel R1 et R4 sont de l'hydrogène.

52. Catalyseur selon la revendication 38, dans lequel n est 4.

53. Catalyseur chiral selon la revendication 38 ayant la formule générale (IVa): 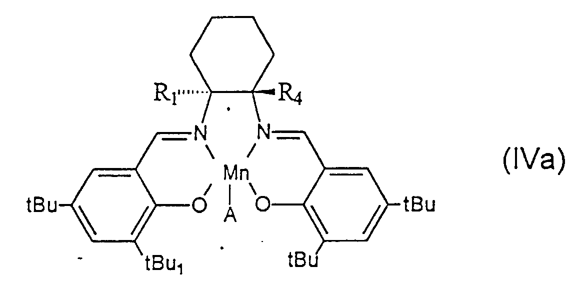
où A est un anion; et
où R1 et R4 sont trans l'un par rapport à l'autre et sont choisis dans le groupe consistant en des alkyles primaires et de l'hydrogène.

54. Catalyseur selon la revendication 53, dans lequel R1 est identique à R4.

55. Catalyseur selon la revendication 54, dans lequel R1 et R4 sont de l'hydrogène.

56. Catalyseur selon la revendication 53, dans lequel R1 et R4 sont choisis dans le groupe consistant en de l'hydrogène et du méthyle.

57. Procédé d'époxydation énantiosélective d'une oléfine prochirale au moyen d'un catalyseur chiral, comprenant les étapes de:
mise à disposition d'une oléfine prochirale;
mise à disposition d'une source d'atomes d'oxygène;
mise à disposition du catalyseur chiral selon la revendication 1, 20, 26, 38 ou 53, et
réaction de ladite oléfine, ladite source d'atomes d'oxygène et ledit catalyseur chiral dans des conditions et pendant une durée suffisantes pour époxyder ladite oléfine.

58. Procédé selon la revendication 57, dans lequel l'oléfine prochirale est choisie dans le groupe consistant en oléfines monosubstituées, cis-1,2-disubstituées et cycliques.

59. Procédé selon la revendication 57, dans lequel l'oléfine prochirale est une oléfine cis-disubstituée portant un substituant primaire sur l'un des côtés de la double liaison et un substituant secondaire, tertiaire ou aryle sur l'autre côté.

60. Procédé selon la revendication 57, dans lequel l'oléfine chirale est choisie dans le groupe consistant en:
cis-β-méthylstyrène, dihydronaphtalène, 2-cyclo-hexényl-l,l-dioxolane, 2,2-diméthylchromène, styrène et propylène.

61. Procédé selon la revendication 57, dans lequel la source d'atomes d'oxygène est choisie dans le groupe consistant en: NaOCl, iodosylmésitylène, NaIO₄, NBu₄IO₄, peroxymonosulfate de potassium, monoperoxyphtalate de magnésium et des ions hexacyanoferrate.

62. Procédé selon la revendication 57, dans lequel la source d'atomes d'oxygène est choisie dans le groupe consistant en: NaOCl et iodosylmésitylène.

63. Procédé selon la revendication 57, dans lequel la source d'atomes d'oxygène est NaOCl.
